# EUROPEAN PATENT SPECIFICATION

(11) **EP 2 165 709 B1**
(45) Date of publication and mention of the grant of the patent: **16.03.2016**
(21) Application number: 08021798.7
(22) Date of filing: 02.08.2004
(51) Int. Cl.: A61K 31/662, A61K 31/63, A61K 31/635, C07C 311/37, C07D 207/44, C07D 213/72, C07D 215/38, C07D 217/22, C07D 233/88, C07D 241/44, C07D 265/30, C07D 401/12, C07D 403/12, C07D 405/12, A61K 45/06, C07F 9/6533, C07C 311/41, C07D 295/215, C07F 9/09

(54) **Pharmaceutical compositions comprising a lysine based compound and an HIV antiviral or antiretroviral agent**
Pharmazeutische Zusammensetzungen enthaltend eine Lysin-basierende Verbindung und ein HIV-antivirales order antiretrovirales Agens
Composition pharmaceutique comprenant un composé à base de lysine et un agent VIH-antiviral ou antirétroviral

(43) Date of publication of application: 24.03.2010
(62) Divisional of application: 04761606.5
(73) Proprietor: Ambrilia Biopharma Inc., Verdun, Quebec H3E 1H4 (CA)
(72) Inventor: Stranix, Brent Richard, Pointe-Claire Québec H9R 3L7 (CA); Perron, Valérie, Laval Quest Québec H7R 6H7 (CA)
(74) Representative: Serjeants LLP

(56) References cited:
- WO-A1-99/33815
- WO-A1-02/064551
- WO-A1-2004/056764
- SOHMA YOUHEI ET AL: "Development of water-soluble prodrugs of the HIV-1 protease inhibitor KNI-727: Importance of the conversion time for higher gastrointestinal absorption of prodrugs based on spontaneous chemical cleavage" JOURNAL OF MEDICINAL CHEMISTRY, AMERICAN CHEMICAL SOCIETY, WASHINGTON, US LNKD- DOI:10.1021/JM030009M, vol. 46, no. 19, 11 September 2003 (2003-09-11), pages 4124-4135, XP002492718 ISSN: 0022-2623 [retrieved on 2003-08-13]
- "FOSAMPRENAVIR" DRUGS OF THE FUTURE, PROUS SCIENCE, ES LNKD- DOI:10.1358/DOF.2001.026.03.615590, vol. 26, no. 3, 1 March 2001 (2001-03-01), pages 224-231, XP009001334 ISSN: 0377-8282
- CHONG K-T ET AL: "PEPTIDOMIMETIC HIV PROTEASE INHIBITORS: PHOSPHATE PRODRUGS WITH IMPROVED BIOLOGICAL ACITIVITIES" JOURNAL OF MEDICINAL CHEMISTRY, AMERICAN CHEMICAL SOCIETY, WASHINGTON, US LNKD- DOI:10.1021/JM00069A018, vol. 36, no. 17, 20 August 1993 (1993-08-20), pages 2575-2577, XP002007120 ISSN: 0022-2623

## Description

### TECHNICAL FIELD OF THE INVENTION

This invention relates to pharmaceutical compositions containing an HIV antiviral or anti-retroviral agent that targets attachment to the cell receptor and cell entry, reverse transcription or viral DNA integration into cellular DNA and one or more lysine based compounds according to general Formula II which present good solubility and bioavailability and which have a physiologically cleavable unit, whereby upon cleavage of the unit the compound is able to release an HIV protease inhibitor. The pharmaceutical compositions of the present invention are particularly well suited for decreasing the pill burden and increasing patient compliance.

Any subject-matter which is not encompassed by the scope of the claims does not form part of the claimed invention.

### BACKGROUND OF THE INVENTION

Inhibitors of the HIV viral protease have been developed relatively recently and their use began only in 1996. Currently, they are considered the most effective drugs against HIV infection. Unfortunately, most current proteases inhibitors are relatively large hydrophobic molecules that possess rather low bioavailability. A high pill burden is therefore required to attain the therapeutic dose in a patient. This is a deterrent, which too often results in patient non-compliance and inadequate treatment results. This situation leads to sub-optimal therapeutic drug concentration that in turns leads to the development of HIV resistant strains. Consequently, there is an urgent need to improve the solubility and bioavailability of proteases inhibitors.

Examples of improved compounds have been developed in the form of prodrugs of aspartyl protease inhibitors such as described, for example, in US patent no. 6,436,989 to Hale et al. This patent shows a novel class of molecules characterized by favourable aqueous solubility, high oral bioavailability and facile *in vivo* generation of the active ingredient. However, it is well known that HIV has the ability to develop resistance to the currently available drugs. Thus, there is a need for alternative HIV protease inhibitors active towards wild-type and resistant viral strains. Thus, molecules derived from current HIV protease inhibitors showing enhanced solubility and bioavailability is desirable to fight resistant viral strains.

A unique class of aromatic derivatives which are inhibitors of aspartyl proteases is described in US patent no. 6,632,816 to Stranix et al.

This patent includes, more particularly, Nε-synthetic amino acid substituted L-lysine derivatives possessing potent aspartyl protease inhibitory properties. However, it would be advantageous to improve these derivatives by enhancing aqueous solubility and bioavailability in order to reduce the pill burden and to favour patient's compliance. Since it is challenging to generate active protease inhibitors, specifically toward wild-type and resistant strains, the formation of derivatives of original HIV protease inhibitors such as inhibitors described in US patent no. 6,632,816 to Stranix et al*,* known to be active toward resistant strains represents a viable route with considerable advantages. More particularly, generation of compounds with enhanced aqueous solubility, bioavailability, time of duration and formulation properties along with other advantages is desirable in the development of an effective drug.

EP 1773763 B1, from which this application is divided, describes and claims, *inter alia,* compounds of Formula II 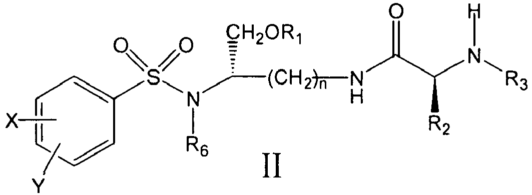 wherein n is 3 or 4,
wherein X and Y, the same or different, are selected from the group consisting of H, a straight alkyl group of 1 to 6 carbon atoms, a branched alkyl group of 3 to 6 carbon atoms, a cycloalkyl group of 3 to 6 carbon atoms, F, C1, Br, I, -CF₃, -OCF₃, -CN, -NO₂, -NR₄R₅,-NHCOR₄, -OR₄, -SR₄, -COOR₄, -COR₄, and -CH₂OH or X and Y together define an alkylenedioxy group selected from the group consisting of a methylenedioxy group of formula -OCH₂O- and an ethylenedioxy group of formula -OCH₂CH₂O-,
wherein R₆ is selected from the group consisting of a straight alkyl group of 1 to 6 carbon atoms, a branched alkyl group of 3 to 6 carbon atoms and a cycloalkylalkyl group having 3 to 6 carbon atoms in the cycloalkyl part thereof and 1 to 3 carbon atoms in the alkyl part thereof,
wherein R₃ is selected from the group consisting of H, a straight alkyl group of 1 to 6 carbon atoms, a branched alkyl group of 3 to 6 carbon atoms, a cycloalkyl group of 3 to 6 carbon atoms, and a group of formula R_{3A}-CO-, R_{3A} being selected from the group consisting of a straight or branched alkyl group of 1 to 6 carbon atoms, a cycloalkyl group having 3 to 6 carbon atoms, a cycloalkylalkyl group having 3 to 6 carbon atoms in the cycloalkyl part thereof and 1 to 3 carbon atoms in the alkyl part thereof, an alkyloxy group of 1 to 6 carbon atoms, tetrahydro-3-furanyloxy, -CH₂OH, -CF₃, -CH₂CF₃, -CH₂CH₂CF₃, pyrrolidinyl, piperidinyl, 4-morpholinyl, CH₃O₂C-, CH₃O₂CCH₂-, Acetyl-OCH₂CH₂-, HO₂CCH₂-, 3-hydroxyphenyl, 4-hydroxyphenyl, 4-CH₃OC₆H₄CH₂-, CH₃NH-, (CH₃)₂N-, (CH₃CH₂)₂N-, (CH₃CH₂CH₂)₂N-, HOCH₂CH₂NH-, CH₃OCH₂O-, CH₃OCH₂CH₂O-, C₆H₅CH₂O-, 2-pyrrolyl, 2-pyridyl, 3-pyridyl, 4-pyridyl-, 2-pyrazinyl, 2-quinolyl, 3-quinolyl, 4-quinolyl, 1-isoquinolyl, 3-isoquinolyl, 2-quinoxalinyl, a phenyl group of formula 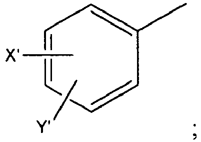 a picolyl group selected from the group consisting of 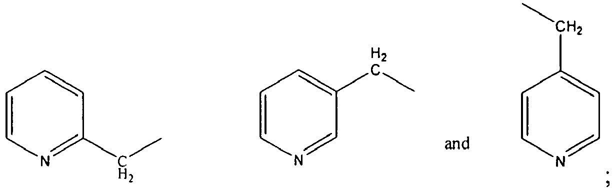 a picolyloxy group selected from the group consisting of 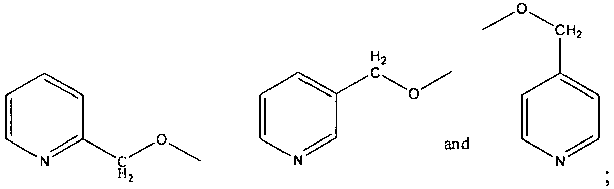 a substituted pyridyl group selected from the group consisting of 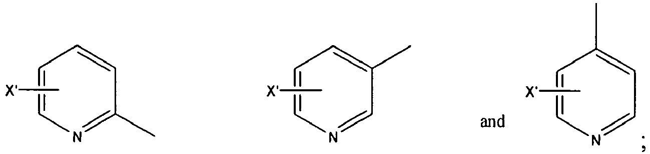 and a group of formula 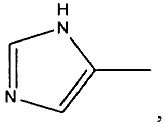 wherein X' and Y', the same or different, are selected from the group consisting of H, a straight alkyl group of 1 to 6 carbon atoms, a branched alkyl group of 3 to 6 carbon atoms, a cycloalkyl group of 3 to 6 carbon atoms, F, Cl, Br, I, -CF₃, -NO₂, -NR₄R₅, -NHCOR₄, -OR₄,-SR₄, -COOR₄, -COR₄ and -CH₂OH,
wherein R₄ and R₅, the same or different, are selected from the group consisting of H, a straight alkyl group of 1 to 6 carbon atoms, a branched alkyl group of 3 to 6 carbon atoms, and a cycloalkyl group of 3 to 6 carbon atoms,
wherein R₂ is selected from the group consisting of a diphenylmethyl group of formula IV, 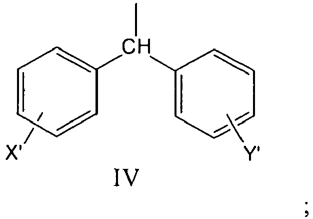 a naphthyl-1-CH₂- group of formula V, 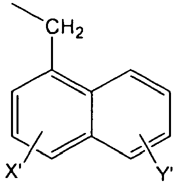 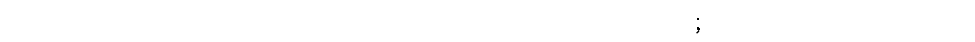 a naphthyl-2-CH₂- group of formula VI, 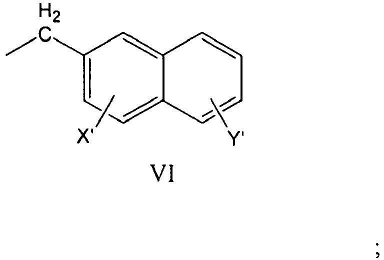 a biphenylmethyl group of formula VII, 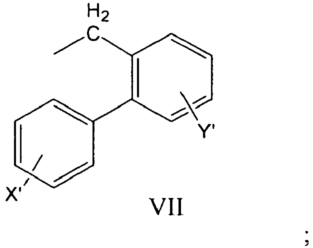 and an anthryl-9-CH₂- group of formula VIII, 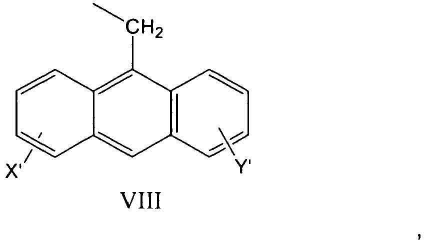 and wherein R₁ is selected from the group consisting of (HO)₂P(O), (MO)₂P(O) and a group of formula R_{1A}-CO-, wherein M is an alkali metal or alkaline earth metal,
wherein R_{1A} is selected from the group consisting of a straight or branched alkyl group of 1 to 6 carbon atoms, a cycloalkyl group having 3 to 6 carbon atoms, a cycloalkylalkyl group having 3 to 6 carbon atoms in the cycloalkyl part thereof and 1 to 3 carbon atoms in the alkyl part thereof, an alkyloxy group of 1 to 6 carbon atoms, -CH₂OH, CH₃O₂C-, CH₃O₂CCH₂-, Acetyl-OCH₂CH₂-, HO₂CCH₂-, 2-hydroxyphenyl, 3-hydroxyphenyl, 4-hydroxyphenyl, (CH₃)₂NCH₂-, (CH₃)₂CHCH(NH₂)-, HOCH₂CH₂NH-, CH₃OCH₂O-, CH₃OCH₂CH₂O-, 2-pyrrolyl, 2-pyridyl, 3-pyridyl, 4-pyridyl, 1-methyl-1,4-dihydro-3-pyridyl, 2-pyrazinyl, 2-quinolyl, 3-quinolyl, 4-quinolyl, 1-isoquinolyl, 3-isoquinolyl, 2-quinoxalinyl, a phenyl group of formula 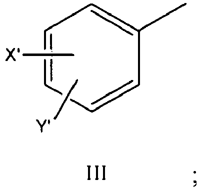 a picolyl group selected from the group consisting of 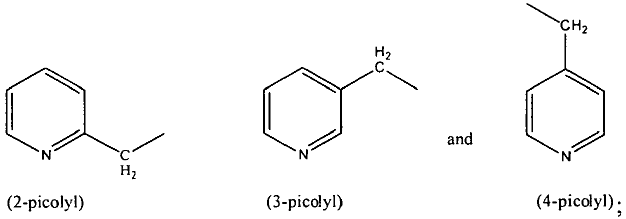 a picolyloxy group selected from the group consisting of 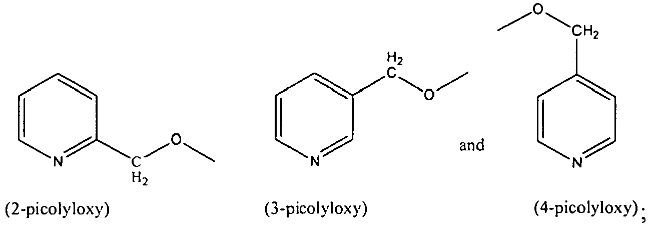 a substituted pyridyl group selected from the group consisting of 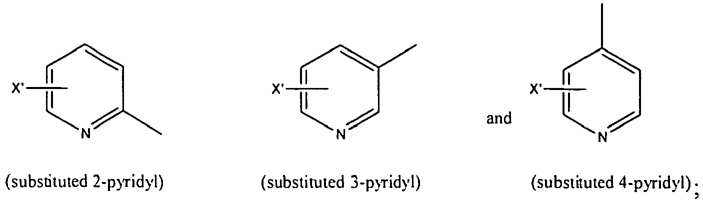 and a group of formula

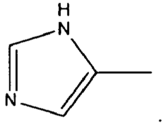

The compounds of Formula II originate from a class of derivatives that are potent aspartyl protease inhibitors. They may readily be cleaved *in vivo* to release the active ingredient. The active ingredient has an affinity for aspartyl proteases, in particular, HIV-1 aspartyl protease (US patent no. 6,632,816). The active ingredients also present potent antiviral activity when tested on non-mutated HIV-1 viral strain (NL4.3 as the wild type virus) as well as several mutant strains. Therefore, the compounds Formula II are useful as a means to increase solubility and improve bioavailability of the active ingredient (protease inhibitor). The compounds of Formula II possess good solubility and bioavailability and may be orally administered as aqueous solution.

### SUMMARY OF THE INVENTION

The present invention provides a pharmaceutical composition comprising an HIV antiviral or anti-retroviral agent that targets attachment to the cell receptor and cell entry, reverse transcription or viral DNA integration into cellular DNA and a compound of Formula II or a pharmaceutically acceptable salt thereof.

In the pharmaceutical composition of the invention, the compound of Formula II preferably has the Formula IIa, IIb or IIc 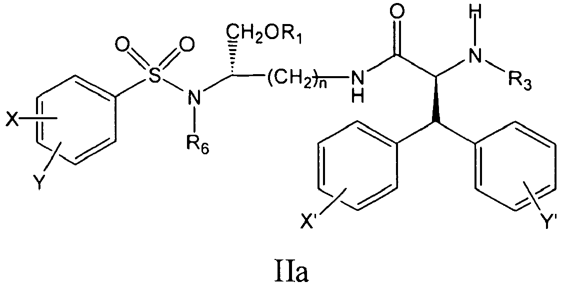 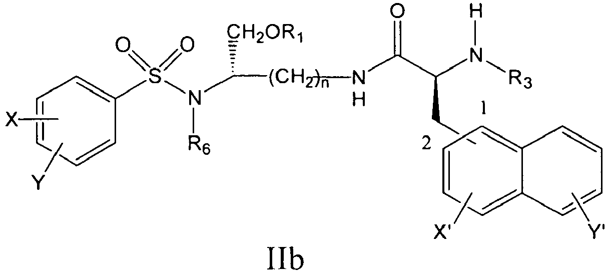 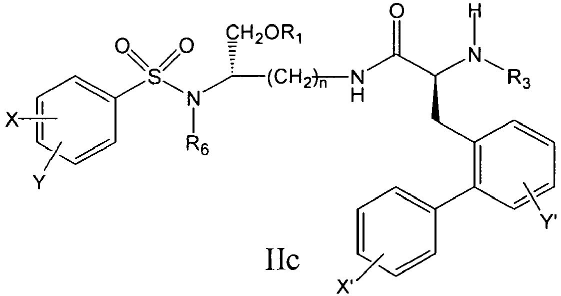 wherein n, X, Y, X', Y', R₁, R₃ and R₆ are as defined herein.

The pharmaceutical compositions of the invention may comprise at least one compound of formula II, IIa, IIb or IIc, or a combination of compounds of formula II, IIa IIb or IIc. The pharmaceutical composition may comprise a pharmaceutically acceptable carrier. The pharmaceutical composition may comprise, for example, a pharmaceutically effective amount of such one or more compounds or as applicable, pharmaceutically acceptable ammonium salts thereof.

For example, the pharmaceutical composition of the present invention may comprise one or more of the following compounds;
- a compound of formula IIa wherein n is 4, R₁ is (HO)₂P(O), X is 4-NH₂, Y is H, X' is H, Y' is H, R₆ is *iso*-butyl and R₃ is CH₃O-CO,
- a compound of formula IIa wherein n is 4, R₁ is (NaO)₂P(O), X is 4-NH₂, Y is H, X' is H, Y' is H, R₆ is *iso*-butyl and R₃ is CH₃O-CO,
- a compound of formula IIa wherein n is 4, R₁ is (HO)₂P(O), X is 4-NH₂, Y is H, X' is H, Y' is H, R₆ is *iso*-butyl and R₃ is CH₃CO,
- a compound of formula IIa wherein n is 4, R₁ is (HO)₂P(O), X is 4-NH₂, Y is 3-F, X' is H, Y' is H, R₆ is *iso*-butyl and R₃ is CH₃O-CO,
- a compound of formula IIa wherein n is 4, R₁ is CH₃CO, X is 4-NH₂, Y is H, X' is H, Y' is H, R₆ is *iso*-butyl and R₃ is CH₃O-CO,
- a compound of formula IIa wherein n is 4, R₁ is 3-pyridyl-CO, X is 4-NH₂, Y is H, X' is H, Y' is H, R₆ is *iso*-butyl and R₃ is CH₃O-CO,
- a compound of formula IIa wherein n is 4, R₁ is (CH₃)₂NCH₂CO, X is 4-NH₂, Y is H, X' is H, Y' is H, R₆ is *iso*-butyl and R₃ is CH₃O-CO,
- a compound of formula IIa wherein n is 4, R₁ is (CH₃)₂CHCH(NH₂)CO, X is 4-NH₂, Y is H, X' is H, Y' is H, R₆ is *iso*-butyl and R₃ is CH₃O-CO,
- a compound of formula IIb wherein n is 4, R₁ is (HO)₂P(O), X is 4-NH₂, Y is H, X' is H, Y' is H, R₆ is *iso*-butyl and R₃ is CH₃O-CO and wherein the naphthyl group is a naphthyl-2-CH₂ group,
- a compound of formula IIb wherein n is 4, R₁ is (HO)₂P(O), X is 4-NH₂, Y is H, X' is H, Y' is H, R₆ is *iso*-butyl and R₃ is 4-morpholine-CO and wherein the naphthyl group is a naphthyl-1-CH₂ group, or
- a combination of any of the above mentioned compounds.

The term "pharmaceutically effective amount" refers to an amount effective in treating or preventing HIV infection in a patient or for reducing or eliminating symptoms of AIDS. It is also to be understood herein that a "pharmaceutically effective amount" may be construed as an amount giving a desired therapeutic effect, either taken into a single or multiple doses or in any dosage or route or taken alone or in combination with other therapeutic agents. In the case of the present invention, a "pharmaceutically effective amount" may be understood as an amount having an inhibitory effect on HIV (HIV-1 and HIV-2 as well as related viruses (e.g., HTLV-I and HTLV-II, and simian immunodeficiency virus (SIV))) infection cycle (e.g., inhibition of replication, reinfection, maturation, budding and on any organism which rely on aspartyl proteases for its life cycle. An inhibitory effect is to be understood herein as an effect such as a reduction in the capacity of an organism (e.g. HIV) to reproduce itself (replicate), to re-infect surrounding cells, or even a complete inhibition (or elimination) of an organism.

The terms "HIV protease" and "HIV aspartyl protease" are used interchangeably and include the aspartyl protease encoded by the human immunodeficiency virus type 1 or 2.

The term "prophylactically effective amount" refers to an amount effective in preventing HIV infection in a patient. As used herein, the term "patient" refers to a mammal, including a human.

The terms "pharmaceutically acceptable carrier", "pharmaceutically acceptable adjuvant" and "physiologically acceptable vehicle" refer to a non-toxic carrier or adjuvant that may be administered to a patient, as part of the pharmaceutical composition of the present invention, and which does not destroy the pharmacological activity thereof.

It is to be understood herein that a "straight alkyl group of 1 to 6 carbon atoms" includes methyl, ethyl, propyl, butyl, pentyl and hexyl.

It is to be understood herein that a "branched alkyl group of 3 to 6 carbon atoms" includes *iso*-butyl, *tert*-butyl, 2-pentyl and 3-pentyl.

It is to be understood herein that a "cycloalkyl group having 3 to 6 carbon" includes cyclopropyl, cyclobutyl, cyclopentyl and cyclocyclohexyl (i.e., C₆H₁₁).

Pharmaceutically acceptable salts of the compounds of Formulae II, IIa, IIb and IIc include those derived from appropriate bases, including alkali metal (e.g., sodium), alkaline earth metal (e.g., magnesium), ammonium and N - (C₁₋₄ alkyl)₄⁺ salts.

Pharmaceutically acceptable salts of the compounds of Formulae II, IIa, IIb and IIc also include those derived from pharmaceutically acceptable inorganic and organic acids and bases. Examples of such acid salts include: acetate, adipate, alginate, aspartate, benzoate, benzenesulfonate, bisulfate, butyrate, citrate, camphorate, camphorsulfonate, cyclopentanepropionate, digluconate, dodecylhydrogensulfate, dodecylsulfate, ethanesulfonate, formate, fumarate, glucoheptanoate, glycerophosphate, glycollate, hemisulfate, heptanoate, hexanoate, hydrochloride, hydrobromide, hydroiodide, 2-hydroxyethanesulfonate, lactate, maleate, malonate, methanesulfonate, 2-naphthylsulfonate, nicotinate, nitrate, oxalate, pamoate, pectinate, perchlorate, persulfate, 3-phenylpropionate, phosphate, picrate, pivalate, propionate, salicylate, succinate, sulfate, tartrate, thiocyanate, tosylate, and undecanoate.

It is also to be understood herein that "g" or "gm" is a reference to the gram weight unit and "C", or "°C" is a reference to the Celsius temperature unit.

The compounds disclosed herein may easily be prepared using conventional techniques from readily available starting materials. The detailed descriptions of these approaches are presented, for example, in schemes 1 to 5 discussed below.

Scheme 1 illustrates a generic example for the preparation of the phosphate monoester ***III*** derived from a primary alcohol (see ***I***), a compound of HIV protease inhibitors (see example 1 (step G and H) in the experimental portion of this document for a specific example of this synthesis).

Note:
a) R₂ and R₃ are as defined herein.

The synthesis of phosphate monoester ***III*** may use a HIV aspartyl protease inhibitor (***I**,* see US patent no. 6,632,816) as the starting material. The diethyl phosphotriester ***II*** was obtained in good yield upon treatment with diethyl chlorophosphate and sodium hydride in a mixture of tetrahydrofuran and triethylphosphate. Then, addition of trimethysilyl bromide in dichloromethane (DCM) gave compound ***III*** in good to excellent yields.

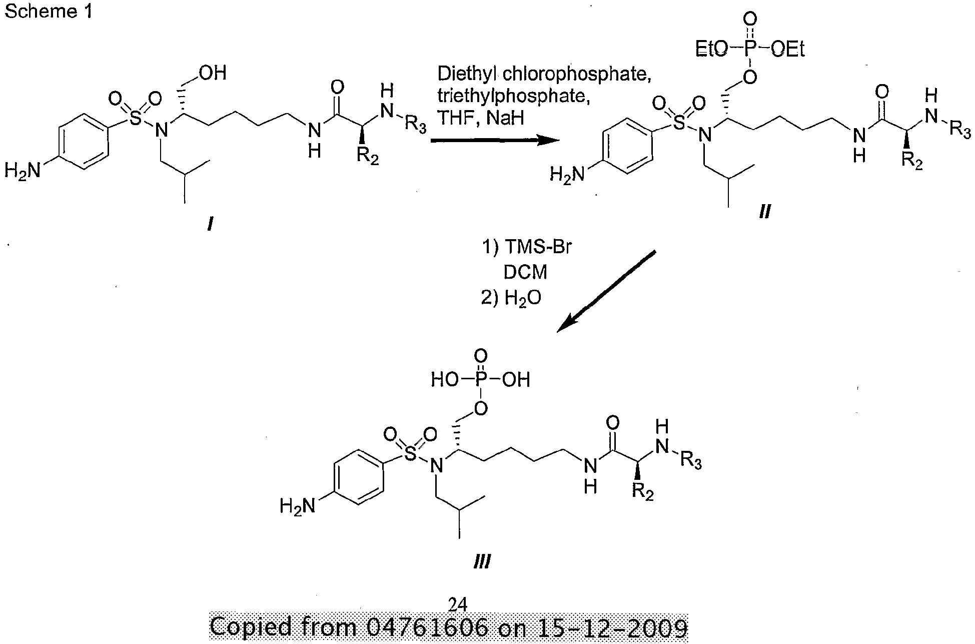

Scheme 1A represents another generic example for the preparation of the phosphate monoester ***IIIA*** derived from a primary alcohol (see ***IA**),* a compound of HIV protease inhibitors.

Note:
a) n, X, Y, R₂, R₃ and R₆ are as defined herein.

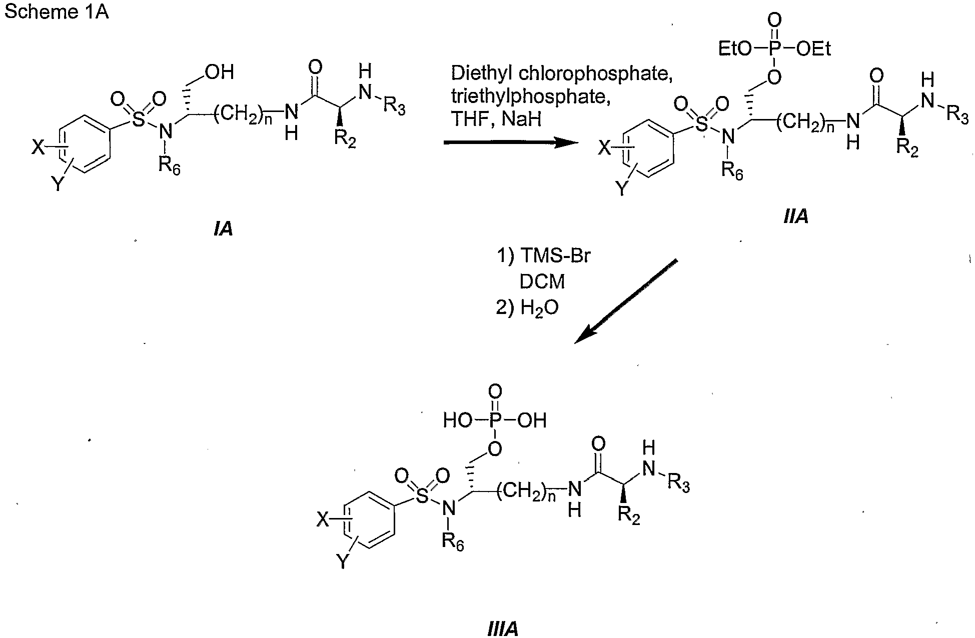

The synthesis of phosphate monoester ***IIIA*** is performed as described for the preparation of ***III*** (scheme 1).

Scheme 2 illustrates a generic example for the preparation of the phosphate monoester ***III**,* a compound of HIV protease inhibitors, with a different approach starting from (3*S*)-3-isobutylamino-azepan-2-one (***IV***).

Note:
a) R₂ and R₃ are as defined herein.

As shown in scheme 2, the phosphate monoester derivative ***III*** was obtained from (3*S*)-3-isobutylamino-azepan-2-one (***IV***) in a seven-step reaction sequence. Initially, (2*S*)-3-isobutylamino-azepan-2-one (***IV***) was sulfonated with 4-acetamidobenzenesulfonyl chloride in the presence of triethylamine in dichloromethane to give compound ***V*** in excellent yields. The derivative ***VI*** was obtained quantitatively upon treatment of ***V*** with di-*tert*-butyl pyrocarbonate and DMAP in acetonitrile. The reductive ring opening with sodium borohydride in ethanol lead to key intermediates ***VII*** in good yield. The diethyl phosphotriester ***VIII*** was obtained in good yield upon treatment with diethyl chlorophosphate and sodium hydride in a mixture of tetrahydrofuran and triethylphosphate. The Boc protective groups were removed upon treatment with HC1 in ethanol to give compound ***IX*** quantitatively (T.W. Greene and P. G. M. Wuts, Protective groups in Organic Synthesis, 3rd Edition, John Wiley & Sons, Inc. 1999). Then, coupling of the free amino group present on intermediate ***IX*** with a variety of synthetic amino acid in the presence of 1-hydroxybenzotriazole (HOBt) and 1-[3-(dimethylamino)propyl]-3-ethylcarbodiimide hydrochloride (EDAC) led to derivative ***II*** in good to excellent yields. Finally, addition of trimethysilyl bromide in dichloromethane (DCM) gave compound ***III*** in good to excellent yields.

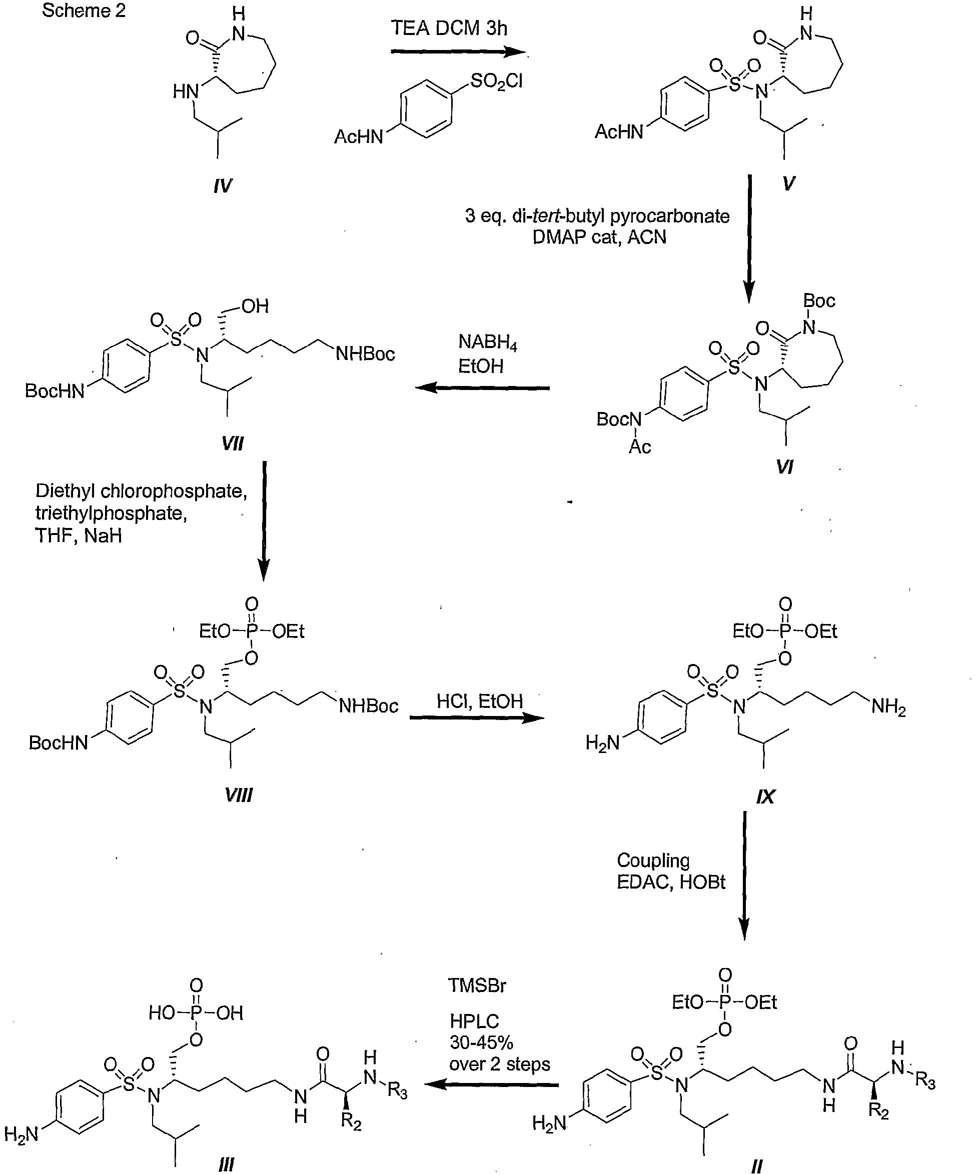

Scheme 3 presents the transformation of a diphenylmethyl derivative; (1*S*,5*S*)-(1-{5-[(4-amino-benzenesulfonyl)-isobutyl-amino]-6-hydroxy-hexylcarbamoyl}-2,2-diphenyl-ethyl)-carbamic acid methyl ester (**PL**-**100**) into its fluorinated phosphate monoester sodium salt analog ***XI**.* This reaction sequence may be used to produce any other similar compounds (compounds) made of unsubstituted (or substituted) diphenylmethyl, 1-naphthyl, 2-naphthyl, biphenyl and 9-anthryl groups described in this invention.

Thus, the treatment of **PL-100** with Selectfluor™ in acetonitrile gave derivative ***X*** in 38% yield. The introduction of the phosphate monoester group was performed as described previously in scheme 1 and 2. First, the diethyl phosphotriester intermediate was obtained in good yield upon treatment with diethyl chlorophosphate and sodium hydride in a mixture of tetrahydrofuran and triethylphosphate. Secondly, addition of trimethysilyl bromide in dichloromethane (DCM) gave the phosphate monoester compound in good to excellent yields. The final product ***XI*** was easily obtained upon treatment of the phosphate monoester with a solution of sodium hydroxide with good yields.

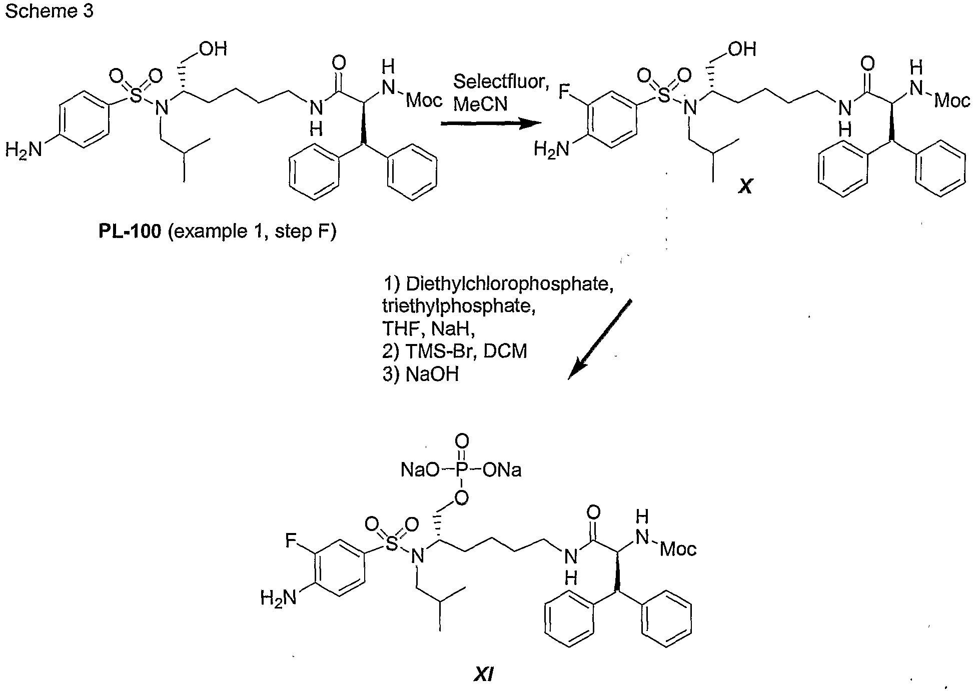

Scheme 4 illustrates a generic example for the transformation of a phosphotriester ***II*** into its fluorinated analog ***XIII*** in a two-step reaction sequence. This generic example represents a second approach for the synthesis of fluorinated compounds of this invention. In this case, the fluorine atom is added to the phosphotriester ***II*** instead of the primary alcohol derivative of general formula ***I*** or, more specifically, **PL-100** as shown on scheme 3. This alternate reaction sequence may be used to produce any other similar compounds made of unsubstituted (or substituted) diphenylmethyl, 1-naphthyl, 2-naphthyl, biphenyl and 9-anthryl groups described in this invention.

Note:
a) R₂ and R₃ are as defined herein.

Briefly, treatment of derivative ***II*** with Selectfluor™ in acetonitrile gave derivative ***XII*** in good yields. Then, addition of trimethysilyl bromide in dichloromethane (DCM) gave the phosphate monoester compound ***XIII*** in good to excellent yields. If desired, the final product ***XIII*** may be easily transformed into the phosphate monoester sodium salt analog as described before in scheme 3.

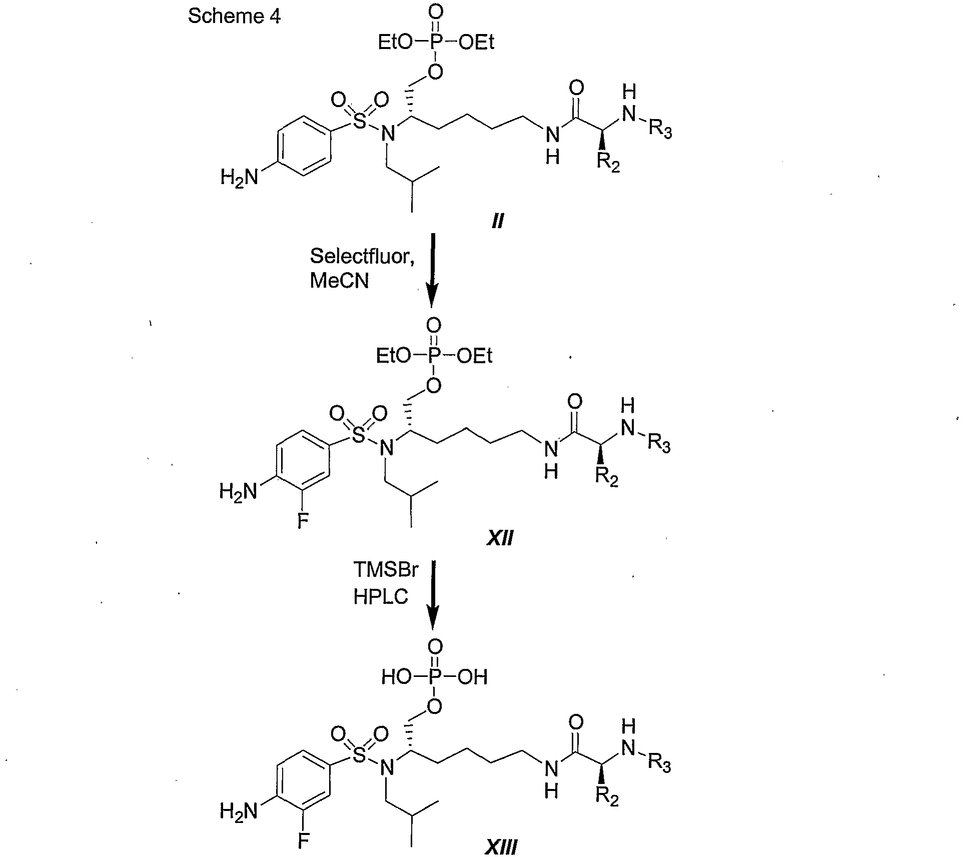

Scheme 5 illustrates the synthesis of various ester compounds ***XVI*** in accordance with the invention. The ester compounds are known to be easily cleaved *in vivo* by esterase enzymes and, as a result, may release the active ingredient. In this scheme R₂ is set as a diphenylmethyl group. However, this reaction sequence may be used to produce any other similar compounds made of unsubstituted (or substituted) diphenylmethyl, 1-naphthyl, 2-naphthyl, biphenyl and 9-anthryl groups described in this invention.

Note:
a) R_{1A} represents the "residue" of the acid molecule that is linked to the free primary alcohol group present on intermediate ***XV*** and is as defined herein.

The compounds ***XVI*** are generally obtained in a three-step reaction sequence in high yields. Esterification of (1*S*)-{4-[(5-tert-butoxycarbonylamino-1-hydroxymethyl-pentyl)-isobutyl-sulfamoyl]-phenyl}-carbamic acid tert-butyl ester (***VII***) with a variety of acid in the presence of 1-hydroxybenzotriazole (HOBt) and 1-[3-(dimethylamino)propyl]-3-ethylcarbodiimide hydrochloride (EDAC) led to the desired esters ***XIV*** in excellent yields. The acetyl ester was obtained quantitatively using acetic anhydride in the presence of *N*,*N-*dimethylaminopyridine (DMAP) in dichloromethane (DCM). Cleavage of the Boc protective group was achieved quantitatively u pon treatment with trifluoroacetic a cid (TFA) i n D CM. A second coupling with (2*S*)-2-methoxycarbonylamino-3,3-diphenyl-propionic acid is performed on the primary amino group of intermediate ***XV*** with HOBt and EDAC to give the desired compounds ***XVI*** in good to excellent yields. If necessary, catalytic hydrogenation of a benzyloxycarbonyl group is performed using 10% palladium on carbon to give the final compound ***XVII**.*

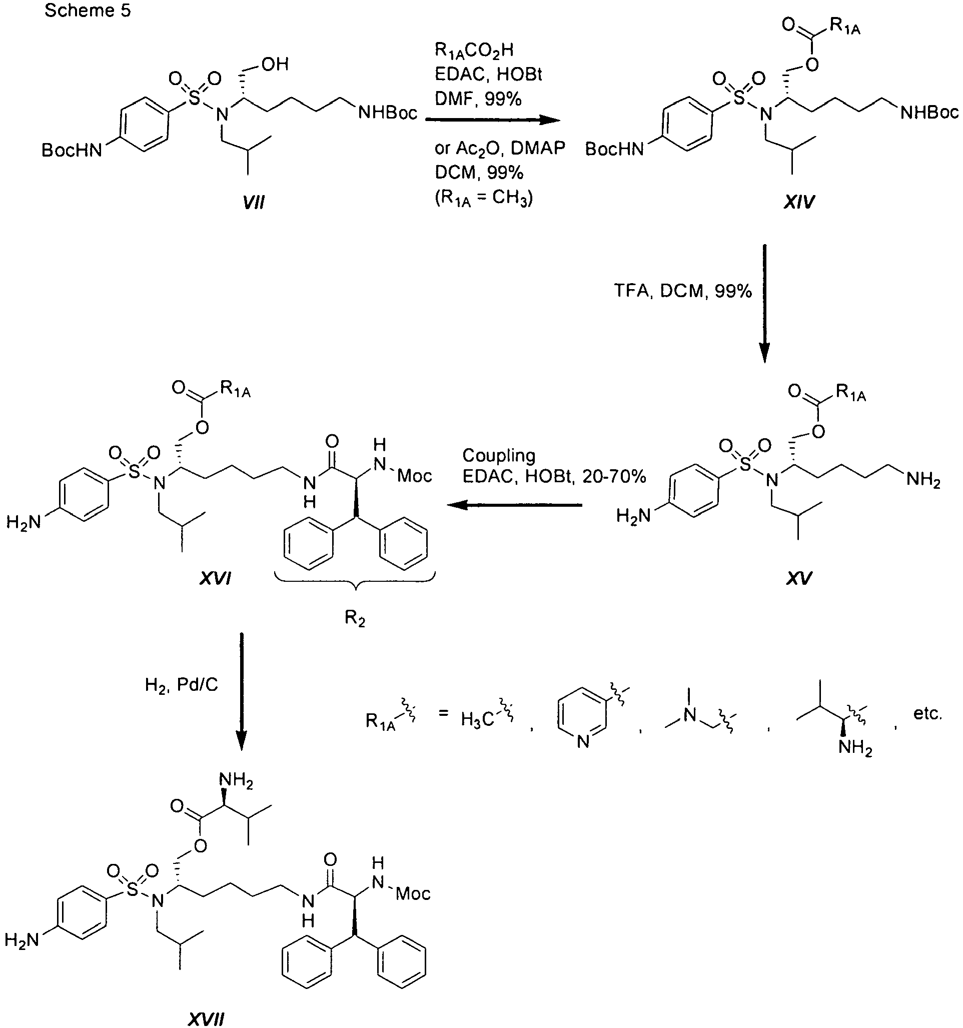

As it may be appreciated by the person skilled in the art, the above synthetic schemes are not intended to be a comprehensive list of all means by which the compound described and claimed in this application may be synthesized but only represent exemplification of synthesis methods among others.

As discussed above, the compounds of Formula II, IIa, IIb, and IIc release the active ingredients that are excellent ligands for aspartyl proteases, for example, HIV-1 protease. Accordingly, these compounds are, by releasing the active ingredient, capable of targeting and inhibiting late stage events in the replication, i.e. the processing of the viral polyproteins by HIV encoded protease. Compounds according to Formula II, IIa, IIb, and IIc advantageously inhibit the ability of the HIV-1 virus to infect immortalized human T cells over a period of days, as determined by an assay measuring the amount of extracellular p24 antigen; a specific marker of viral replication (see, Meek et al., Nature, 343, pp. 90-92 (1990)).

In addition to their use in the prophylaxis or treatment of HIV or HTLV infection, the compounds according to Formula II, IIa, IIb, and IIc may also be used as inhibitory or interruptive agents for other viruses which use aspartyl proteases, similar to HIV or HTLV aspartyl proteases, in their life cycle. Such compounds inhibit the proteolytic processing of viral polyprotein precursors by inhibiting aspartyl protease. Because aspartyl protease is essential for the production of mature virions, inhibition of that processing effectively blocks the spread of virus by inhibiting the production and reproduction of infectious virions, particularly from acutely and chronically infected cells. The compounds of Formula II, IIa, IIb, and IIc advantageously inhibit aspartyl proteases, thus blocking the ability of aspartyl proteases to catalyze the hydrolysis of peptide bonds.

The compounds of Formula II, IIa, IIb, and IIc may be employed in a conventional manner for the treatment or prevention of HIV, HTLV, and other viral infections, which involve aspartyl proteases for their life (replication) cycle. Such methods of treatment, their dosage levels and requirements may be selected by those of ordinary skill in the art from available methods and techniques. For example, a compound of Formula II, IIa, IIb and IIc may be combined with a pharmaceutically acceptable adjuvant for administration to a virally infected patient in a pharmaceutically acceptable manner and in an amount effective to lessen the severity of the viral infection.

Alternatively, the compounds of Formula II, IIa, IIb, and IIc may be used in vaccines and methods for protecting individuals against viral infection over an extended period of time. The compounds may be employed in such vaccines either alone or together with other compounds of this invention in a manner consistent with the conventional utilization of protease inhibitors or protease inhibitors derivatives in vaccines. For example, a compound of Formula II, IIa, IIb, and IIc may be combined with pharmaceutically acceptable adjuvants, or delivery systems conventionally employed in vaccines and administered in prophylactically effective amounts to protect individuals over an extended period of time against viral infections, such as HIV infection. As such, the compounds of Formula II, IIa, IIb, and IIc (upon cleavage of a physiologically cleavable unit) may be administered as agents for treating or preventing viral infections, including HIV infection, in a mammal.

The compounds of Formula II, IIa, IIb, and IIc may be administered to a healthy or HIV-infected patient (before or after the onset of AIDS symptoms) either as a single agent or in combination with other antiviral agents which interfere with the replication cycle of HIV. By administering the compounds of this invention with other antiviral agents which target different events in the viral life cycle, the therapeutic effect of these compounds is potentiated. For instance, the co-administered antiviral agent may be one which targets early events in the viral life cycle, such as attachment to the cell receptor and cell entry, reverse transcription and viral DNA integration into cellular DNA. Antiviral agents targeting such early life cycle events include among others polysulfated polysaccharides, sT4 (soluble CD4) and other compounds which block binding of virus to CD4 receptors on CD4 bearing T-lymphocytes and other CD4(+) cells, or inhibit fusion of the viral envelope with the cytoplasmic membrane, and didanosine (ddI), zalcitabine (ddC), stavudine (d4T), zidovudine (AZT) and lamivudine (3TC) which inhibit reverse transcription. For example another protease inhibitor may be used with compounds of Formula II, IIa, IIb, and IIc. Other anti-retroviral and antiviral drugs may also be co-administered with the compounds of this invention to provide therapeutic treatment for substantially reducing or eliminating viral infectivity and the symptoms associated therewith. Examples of other antiviral agents include ganciclovir, dideoxycytidine, trisodium phosphonoformate, eflornithine, ribavirin, acyclovir, *alpha* interferon and trimenotrexate. Additionally, other types of drugs may be used to potentiate the effect of the compounds of Formula II, IIa, IIb, and IIc, such as viral uncoating inhibitors, inhibitors of Tat or Rev trans-activating proteins, antisense molecules or inhibitors of the viral integrase. These compounds may also be co-administered with other inhibitors of HIV aspartyl protease. Furthermore, it may be found useful to administer compounds of Formula II, IIa, IIb, and IIc with any other drug (other anti-viral compounds, antibiotics, pain killer, etc.,).

Combination therapies with pharmaceutical compositions according to this invention exert a synergistic effect in inhibiting HIV replication because each component agent of the combination acts on a different site of HIV replication. The use of such combinations also advantageously reduces the dosage of a given conventional anti-retroviral agent that would be required for a desired therapeutic or prophylactic effect as compared to when that agent is administered as a monotherapy. These combinations may reduce or eliminate the side effects of conventional single anti-retroviral agent therapies while not interfering with the anti-retroviral activity of those agents. These combinations reduce the potential of resistance to single agent therapies, while minimizing any associated toxicity. These combinations may also increase the efficacy of the conventional agent without increasing the associated toxicity. Combination therapies encompassed by the present invention include, for example, the administration of a compound of Formula II, IIa, IIb, and IIc with AZT, 3TC, ddI, ddC, d4T or other reverse transcriptase inhibitors.

Alternatively, the compounds of Formula II, IIa, IIb, and IIc may also be co-administered with other HIV protease inhibitors such as Ro 31-8959 (Saquinavir; Roche), L-735,524 (Indinavir; Merck), AG-1343 (Nelfinavir; Agouron), A-84538 (Ritonavir; Abbott), ABT-378/r (Lopinavir; Abbott), and VX-478 (Amprenavir; Glaxo) to increase the effect of therapy or prophylaxis against various viral mutants or members of other HIV quasi species.

Administration of compounds of Formula II, IIa, IIb, and IIc may be performed, for example, as single agents or in combination with retroviral reverse transcriptase inhibitors, or other HIV aspartyl protease inhibitors. Co-administration of the compounds of Formula II, IIa, IIb, and IIc with retroviral reverse transcriptase inhibitors or HIV aspartyl protease inhibitors may exert a substantial synergistic effect, thereby preventing, substantially reducing, or completely eliminating viral infectivity and its associated symptoms.

The compounds of Formula II, IIa, IIb, and IIc may be administered in such a manner or form which may allow cleavage of the R₁ unit to release a protease inhibitor. The compounds of Formula II, IIa, IIb, and IIc may also be administered, for example, in combination with immunomodulators (e.g., bropirimine, anti-human *alpha* interferon antibody, IL-2, GM-CSF, methionine enkephalin, interferon *alpha,* diethyldithiocarbamate sodium, tumor necrosis factor, naltrexone and rEPO) antibiotics (e.g., pentamidine isethionate) or vaccines to prevent or combat infection and disease associated with HIV infection, such as AIDS and ARC.

When the pharmaceutical compositions comprising the compounds of Formula II, IIa, IIb, and IIc are administered in combination therapies with other agents, they may be administered sequentially or concurrently to the patient. Alternatively, pharmaceutical or prophylactic compositions according to this invention may be comprised of a combination of one or more compounds of this invention and another therapeutic or prophylactic agent.

Although this invention focuses on the use of the pharmaceutical compositions disclosed herein for preventing and treating HIV infection, the compounds of this invention may also be used as inhibitory agents for other viruses that depend on similar aspartyl proteases for obligatory events in their life cycle. These viruses include retroviruses causing AIDS-like diseases such as simian immunodeficiency viruses, HIV-2, HTLV-I and HTLV-II. In addition, the compounds of this invention may also be used to inhibit other aspartyl proteases and, in particular, other human aspartyl proteases including renin and aspartyl proteases that process endothelin precursors.

Pharmaceutical compositions of this invention comprise any of the compounds of Formula II, IIa, IIb, and IIc, and pharmaceutically acceptable salts thereof, with any pharmaceutically acceptable carrier, adjuvant or vehicle. Pharmaceutically acceptable carriers, adjuvants and vehicles that may be used in the pharmaceutical compositions of this invention include ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethyleneglycol, sodium carboxymethylcellulose, polyacrylates, waxes, polyethylene-polyoxypropylene-block polymers, polyethylene glycol and wool fat.

The pharmaceutical compositions of this invention may be administered orally, parenterally by inhalation spray, topically, rectally, nasally, buccally, vaginally or via an implanted reservoir. It is therefore understood herein that oral administration or administration by injection are encompassed by the present invention. For example, pharmaceutical compositions of the present invention, may, for example, be orally administered in an aqueous solution. The pharmaceutical compositions of this invention may contain any conventional non-toxic pharmaceutically acceptable carriers, adjuvants or vehicles. The term "parenteral" as used herein includes subcutaneous, intracutaneous, intravenous, intramuscular, intra-articular, intrasynovial, intrasternal, intrathecal, intralesional and intracranial injection or infusion techniques.

The pharmaceutical compositions may be in the form of a sterile injectable preparation, for example, as a sterile injectable aqueous or oleaginous suspension. This suspension may be formulated according to techniques known in the art using suitable dispersing or wetting agents (such as, for example, Tween 80) and suspending agents. The sterile injectable preparation may also be a sterile injectable solution or suspension in a non-toxic parenterally acceptable diluent or solvent, for example, as a solution in 1,3-butanediol. Among the acceptable vehicles and solvents that may be employed are amino acid, water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically-acceptable oils, such as olive oil or castor oil, especially in their polyoxyethylated versions. These oil solutions or suspensions may also contain a long-chain alcohol diluent or dispersant, such as Ph. Helv. or a similar alcohol.

The pharmaceutical compositions of this invention may be orally administered in any orally acceptable dosage form including capsules, tablets, and aqueous suspension and solutions. In the case of tablets for oral use, carriers that are commonly used include lactose and corn starch. Lubricating agents, such as magnesium stearate, are also typically added. For oral administration in a capsule form, useful diluents include lactose and dried cornstarch. When aqueous suspensions are administered orally, the active ingredient is combined with emulsifying and suspending agents. If desired, certain sweetening and/or flavoring and/or coloring agents may be added.

The pharmaceutical compositions of this invention may also be administered in the form of suppositories for rectal administration. These compositions may be prepared by mixing a compound of this invention with a suitable non-irritating excipient which is solid at room temperature but liquid at the rectal temperature and therefore will melt in the rectum to release the active components. Such materials include cocoa butter, beeswax, and polyethylene glycols.

Topical administration of the pharmaceutical compositions of this invention is especially useful when the desired treatment involves areas or organs readily accessible by topical application. For application topically to the skin, the pharmaceutical composition should be formulated with a suitable ointment containing the active components suspended or dissolved in a carrier. Carriers for topical administration of the compounds of this invention include, but are not limited to, mineral oil, liquid petroleum, white petroleum, propylene glycol, polyoxyethylene or polyoxypropylene compound, emulsifying wax and water. Alternatively, the pharmaceutical compositions may be formulated with a suitable lotion or cream containing the active compound suspended or dissolved in a carrier. Suitable carriers include, but are not limited to, mineral oil, sorbitan monostearate, polysorbate 60, cetyl esters wax cetearyl alcohol, 2-octyldodecanol, benzyl alcohol and water. The pharmaceutical compositions of this invention may also be topically applied to the lower intestinal tract by rectal suppository formulation or in a suitable neat formulation. Topically-transdermal patches are also included in this invention.

The pharmaceutical compositions of this invention may be administered by nasal aerosol or inhalation. Such compositions are prepared according to techniques well-known in the art of pharmaceutical formulation and may be prepared as solutions in saline employing benzyl alcohol or other suitable preservatives, absorption promoters to enhance bioavailability, fluorocarbons, and/or other solubilizing or dispersing agents known in the art.

Dosage levels of between 0.01 and 25 mg/kg body weight per day, for example from between 0.5 and 25 mg/kg body weight per day of the active ingredient compound are useful in the prevention and treatment of viral infection, including HIV infection. Typically, the pharmaceutical compositions of this invention will be administered from 1 to 5 times per day or alternatively, as a continuous infusion. Such administration may be used as a chronic or acute therapy. The amount of active ingredient that may be combined with the carrier materials to produce a single dosage form will vary depending upon the patient treated and the particular mode of administration. A typical preparation will contain from 5% to 95% active compound (w/w). For example, such preparations may contain from 20% to 80% active compound.

Upon improvement of a patient's condition, a maintenance dose of a composition or combination of this invention may be administered if necessary. Subsequently, the dosage or frequency of administration, or both, may be reduced, as a function of the symptoms, to a level at which the improved condition is retained. When the symptoms have been alleviated to the desired level, treatment should cease. Patients may, however, require intermittent treatment on a long-term basis, upon any recurrence of disease symptoms.

As the person skilled in the art will appreciate, lower or higher doses than those recited above may be desired. Specific dosage and treatment regimen for any particular patient may depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician.

In the description herein, the following abbreviations are used:

| **Abbreviation** | **Meaning** |
|---|---|
| Ac | Acetyl |
| AcOH | Acetic acid |
| APCI | Atmospheric pressure chemical ionization |
| AIDS | Acquired Immunodeficiency Syndrome |
| AZT | 3-Azido-3-deoxythymine (Zidovudine) |
| Boc | Benzyloxycarbonyl |
| t-Butyl | *tert-*Butyl |
| CAM | Cerium ammonium molybdate |
| DCM | Dichloromethane |
| DMAP | *N,N*-dimethylaminopyridine |
| DMSO | Dimethylsulfoxide |
| DMF | Dimethylformamide |
| DNA | Deoxyribonucleic acid |
| EDAC | 1-[3-(dimethylamino)propyl]-3-ethylcarbodiimide hydrochloride |
| EtOAc | Ethyl acetate |
| EtOH | Ethyl alcohol |
| g | Gram |
| h | hour |
| HIV-1, -2 | Human immunodeficiency virus type 1, type 2 |
| HOBt | 1-Hydroxybenzotriazole |
| HPLC | High performance liquid chromatography |
| HTLV-I, -II | Human T-cell lymphotropic virus type I, type II |
| IL-2 | Interleukin-2 |
| Kg | Kilogram |
| L | Liter |
| LC-MS | Liquid chromatography-mass spectrometry |
| M | Molar |
| MeOH | Methyl alcohol |
| mg | Milligram |
| mp | Melting point |
| min | Minute |
| Moc | Methoxycarbonyl |
| mol | Mole |
| mL | Milliliter |
| mmol | Millimole |
| nm | Nanometer |
| nM | Nanomolar |
| po | Orally |
| rEPO | Recombinant erythropoietin |
| TLC | Thin layer chromatography |
| 3TC | 2',3'-Dideoxy-3-thiacytidine |
| TFA | Trifluoroacetic acid |
| THF | Tetrahydrofuran |

### EXAMPLES

This section describes the synthesis of lysine based compounds able to release an HIV aspartyl protease inhibitors as described herein. These examples are for the purpose of illustration only.

### Materials and Methods

Analytical thin layer chromatography (TLC) was carried out with 0.25 mm silica gel E. Merck 60 F₂₅₄ plates and eluted with the indicated solvent systems. Preparative chromatography was performed by flash chromatography, using silica gel 60 (EM Science) with the indicated solvent systems and positive air pressure to allow proper rate of elution. Detection of the compounds was carried out by exposing eluted plates (analytical or preparative) to iodine, UV light and/or treating analytical plates with a 2% solution of *p-*anisaldehyde in ethanol containing 3% sulfuric acid and 1% acetic acid followed by heating. Alternatively, analytical plates may be treated with a 0.3% ninhydrin solution in ethanol containing 3% acetic acid and/or a CAM solution made of 20 g (NH₄)₆Mo₇O₂₄ and 8.3 g Ce(SO₄)₂ polyhydrate in water (750 mL) containing concentrated sulfuric acid (90 mL).

Preparative HPLC were performed on a Gilson apparatus equipped with a C18 column, a 215 liquid handler module and 25 mL/min capacity head pumps. The HPLC is operated with a Gilson UniPoint System Software.

### Semi-preparative HPLC conditions for purification of test compounds:

HPLC system: 2 Gilson #305-25 mL pumps, Gilson #215 liquid handler for injection and collection and a Gilson #155 UV-Vis absorbance detector, all controlled from a Gilson Unipoint V1.91 software
Column: Alltech (#96053) Hyperprep PEP, C-18, 100 Åα, 8 µm, 22 x 250 mm
Flow: 15 mL/min
Solvents: A: H₂O; B: CH₃CN
Gradient: 25% to 80% of B over 40 min
Detector: absorbance; λ: 210 & 265 nm

The crude material dissolved in acetonitrile to a concentration of around 50 to 80 mg / 2 mL were injected in each run. Fractions were collected in amounts of 9 mL pertaining absorbance was detected at the UV detector.

Unless otherwise indicated, all starting materials were purchased from a commercial source such as Aldrich Co. or Sigma Co.

Melting points (mp) were determined on a Büchi 530 melting point apparatus in capillary tubes and were uncorrected.

Mass spectra were recorded on a Hewlett Packard LC/MSD 1100 system using APCI or electrospray sources either in negative mode or positive mode.

Nuclear magnetic resonance (NMR) spectra were recorded on a Bruker AMX-II-500 equipped with a reversed or QNP probe. Samples were dissolved in deuterochloroform (CDCl₃), deuteroacetone (acetone-d₆), deuteromethanol (CD₃OD) or deuterodimethylsulfoxide (DMSO-d₆) for data acquisition using tetramethylsilane as internal standard. Chemical shifts (*) are expressed in parts per million (ppm), the coupling constants (*J*) are expressed in hertz (Hz) whereas multiplicities are denoted as s for singlet, d for doublet, 2d for two doublets, dd for doublet of doublets, t for triplet, q for quartet, quint. for quintet, m for multiplet, and br s for broad singlet.

### DETAILED DESCRIPTION OF THE INVENTION

### EXAMPLES:

### Specific examples for the preparation of derivatives of general formula I

The following compounds were prepared from L-lysine derivatives using the procedures summarized in schemes 1, 1A, 2, 3, 4 and 5 of this invention.

### Example 1. Preparation of (1S,5S)-(1-{5-[(4-amino-benzenesulfonyl)-isobutyl-amino]-6-phosphonooxy-hexylcarbamoyl}-2,2-diphenyl-ethyl)-carbamic acid methyl ester (PL-461)

The preparation of the title compound is based on schemes 1 and 2 of this invention.

### Step A. Preparation of (3S)-3-isobutylamino-azepan-2-one (IV)

L-*α*-amino-,-caprolactam (22.0 g) was dissolved in cold dichloroethane (DCM, 200 mL). isobutyraldehyde (12.6 g) was added slowly and stirred until the heat evolved was dissipated (water forms at the surface). The cold solution was added to 46.5 g of powdered NaBH(OAc)₃ in DCM (0.5 L). AcOH (70 mL) was added to the solution. The slightly turbid mixture was stirred at 20 °C for 4 h. A 500 mL solution of 2M NaOH was added slowly to the turbid mixture and the pH adjust to 11 using a concentrated NaOH solution; and then the mixture stirred for a further 20 min. After extraction, the DCM layer was dried with MgSO₄, filtered and evaporated. The oil thus obtained crystallizes slowly on standing (27.8 g, 85%) and was used without further purification in the next step.
¹H NMR (CDCl₃): *δ* 0.93 (d, *J =* 6.5, 3H), 0.97 (d, *J* = 6.5, 3H), 1.39 (t, *J =* 9.8, 1H), 1.47 (m, 1H), 1.78-1.65 (m, 2H), 2.00-1.93 (m, 2H), 2.32-2.2 (m, 2H), 2.38 (t, *J* = 9.7, 1H), 3.16 (m, 3H), 6.62 (s, 1H (NH)). mp 52-54 °C (hexanes).
A small sample was converted to the *S*-methyl benzyl urea by adding the solid to a solution of *S*-methyl benzyl isocyanate in MeCN. NMR gives 98% ee

### Step B. Preparation of Nα-isobutyl-Nα-(4-acetamidobenzenesulfonyl)-L-α-amino-,-caprolactam (V)

*Nα*-isobutyl-L-*α*-amino-,-caprolactam (***IV***) (4.1 g free base) was dissolved in DCM (200 mL) and treated with 4.0 g triethylamine, followed by 4-acetamidobenzenesulfonyl chloride (5.2 g). A 0.1 g portion of dimethylaminopyridine was added and the mixture was stirred 5 h. The resulting thick slurry was poured into 500 mL 0.5 M HCl and shaken vigorously. The solid in the biphasic solution was filtered out and washed with cold acetone to give 7.3 g (87%) of clean product.
¹H NMR (DMSO-*d*₆): * 0.93 (d, *J* = 6.0, 3H), 0.96 (d, *J* = 6.0, 3H), 1.39 (t, *J* = 12.0, 1H), 1.85-1.65 (m, 3H), 2.08-2.18 (m and s, 6H), 2.90-2.97 (m, 1H), 3.00-3.06 (m, 2H), 3.35 (dd, *J* = 14.2, 8.5, 1H), 4.65 (d, J = 8.7,1H), 6.3 (s, 1H), 7.42 (d, *J* = 8.8, 2H), 7.6 (d, *J* = 8.8, 2H). mp 230-233 °C (EtOH).

### Step C. Preparation of (3S)-3-{[4-(acetyl-tert-butoxycarbonyl-amino)-benzenesulfonyl]-isobutyl-amino}-2-oxo-azepane-1-carboxylic acid tert-butyl ester (Boc activation) (VI)

4.2 g of *N*α-isobutyl-*N*α-(4-acetamidobenzenesulfonyl)-L-*α*-amino-,-caprolactain (***V***) was suspended in 30 mL MeCN and briefly sonicated to break up any large chunks. To this white suspension was added 6.7 g (3 eq.) of di-*tert*-butyl pyrocarbonate in 10 mL MeCN. The suspension was stirred with a magnetic bar and a 120 mg portion of DMAP was added. The solution becomes a clear light yellow after a few minutes. TLC (EtOAc) reveals 1 product Rf 0.9 (starting material Rf at 0.4). The solution is poured in distilled water 20 mL and extracted with ether, dried with Na₂SO₄ and evaporated yielding 6.90 g. A sample was recrystallized from hexanes.
¹H NMR (DMSO-*d*₆): * 0.68 (d, *J* = 6.0, 3H), 0.85 (d, *J* = 6.0, 3H), 1.39 (s, 10H), 1.47 (s, 9H), 1.85-1.65 (m, 3H), 2.15 (s, 3H), 2.80 (q, *J=* 4, 1H), 3.10-3.36 (m, 2H), 4.01 (d, *J=* 8.0, 1H), 4.85 (d, *J=* 8.7, 1H), 7.32 (d, *J* = 8.8, 2H), 7.87 (d, *J=* 8.8, 2H). mp 123-124 °C

### Step D. Preparation of -(1S)-4-amino-N-(5-amino-1-hydroxymethyl-pentyl)-N-isobutyl-benzenesulfonamide (VII-deprotected) (reductive ring opening and deprotection)

A 3.0 g portion of (3*S*)-3-{[4-(acetyl-tert-butoxycarbonyl-amino)-benzenesulfonyl]-isobutyl-amino}-2-oxo-azepane-1-carboxylic acid tert-butyl ester (***VI**,* step C) is dissolved in 40 mL EtOH followed by 750 mg NaBH₄. Brief heating with a heat gun gives a clear solution. TLC reveals one streaky spot after 20 min (EtOAc). The solution is concentrated to a paste, poured in 40 mL 1N NaOH and extracted with ethyl acetate, the organic phase dried with NaSO₄ and evaporated to give 2.8 g of product intermediate (***VII***); (1*S*)-{4-[(5-tert-butoxycarbonylamino-1-hydroxymethyl-pentyl)-isobutyl-sulfamoyl]-phenyl}-carbamic acid tert-butyl ester (***VII***)*.*

The above product intermediate is dissolved in 5 mL EtOH and 5 mL 12 N HCl is added. Vigorous gas evolution is observed for a few minutes. After 2 h the solution is evaporated and rendered basic with concentrated KOH and extracted with EtOAc yielding 1.75 g of a white powder.
¹H NMR (DMSO-*d*₆): * 0.82 (m, 6H), 0.97-1.12 (m, 2H), 1.15-1.30 (m, 3H), 1.57 (m, 1H), 1.84 (m, 1H), 2.40 (t, *J* = 7.8, 2H), 2.75 (m, 1H), 2.85 (m, 1H), 3.21 (m, 1H), 3.44 (d, *J* = 6.4, 2H), 5.92 (br s, 2H), 6.59 (d, *J=* 8.0, 2H), 7.39 (d, *J* = 8.0, 2H).

### Step E. Preparation (2S)-2-methoxycarbonylamino-3,3-diphenyl-propionic acid

To a solution of L-diphenylalanine (241 mg, 1.0 mmol) (Peptech Corp.) in 5 mL 1N NaOH and 0.5 mL saturated Na₂CO₃ (resulting solution at pH 10) was added methoxycarbonyloxysuccinimide (carbonic acid 2,5-dioxo-pyrrolidin-1-yl ester methyl ester) (180 mg, 1.1 mmol) dissolved in 5 mL. Afterwards, the reaction mixture was stirred at room temperature for 2 h. The alkaline solution was extracted once with ether (10 mL) and the aqueous phase was acidified with 1N HCl. This was extracted twice with 20 mL EtOAc, and the combined organic phases were washed with 50 mL 1N HCl. The organic phase was dried over Na₂SO₄, filtered and evaporated to an oil, which solidifies to yields 250 mg (83%) of the desired material. This derivative was used as such in the next step.

### Step F. Preparation of (1S,5S)-(1-{5-[(4-amino-benzenesulfonyl)-isobutyl-amino]-6-hydroxy-hexylcarbamoyl}-2,2-diphenyl-ethyl)-carbamic acid methyl ester (PL-100)

The title compound was prepared from (1*S*)-4-amino-*N*-(5-amino-1-hydroxymethyl-pentyl)-*N*-isobutyl-benzenesulfonamide (***VII*-deprotected**) (step D) and (2*S*)-2-methoxycarbonylamino-3,3-diphenyl-propionic acid (step E) using the coupling procedure with HOBt and EDAC described in example 3 (step D). The final product was obtained in 67% yield (121 mg).
LC-MS : 625.3 (M+H)⁺, 95% pure
¹H NMR (CD₃OD): *δ* 0.71-0.85 (m, 2H), 0.88 (d, *J* = 6.3*,* 5H), 0.91-0.96 (m, 2H), 1.29-1.34 (m, 1H), 1.41-1.52 (m, 1H) 1.82-1.92 (m, 1H), 2.61-2.68 (m, 1H), 2.81-2.85 (m, 2H), 2.94-3.05 (m, 2H), 3.38-3.40 (t, *J* = 5.0, 1H), 3.50-3.51 (m, 1H), 3.52 (s, 3H), 4.28 (d, *J* = 11.0 1H), 4.87 (d, *J* = 11.0, 1H), 6.69 (d, *J =* 8.0, 2H), 7.15-718 (m, 2H), 7.20-7.31 (m, 6H), 7.33 (d, *J =* 7.9, 2H), 7.47 (d, *J =* 7.5, 1H).
¹³C NMR (CD₃OD): *δ* 20.0, 20.1, 23.3, 25.4, 28.1, 28.5, 28.9, 38.1, 40.0, 51.2, 51.6, 53.1, 57.2, 57.4, 59.5, 61.9, 62.4, 112.6, 125.7, 126.2, 126.3, 127.9, 128.1,128.15, 128.2, 128.4, 128.7, 141.3, 141.9, 152.4, 155.9, 169.9, 172.5.

### Step G. Preparation of (1S,5S)-{1-[5-[(4-amino-benzenesulfonyl)-isobutyl-amino]-6-(diethoxy-phosphoryloxy)-hexylcarbamoyl]-2,2-diphenyl-ethyl}-carbamic acid methyl ester

The PL-100 compound (product of step F, 203 mg, 0.325 mmol) was dissolved in dry tetrahydrofuran (3 mL) and 0.2 mL triethylphosphate under N₂ atmosphere. The mixture was stirred at this temperature for 15 min, followed by the addition of diethyl chlorophosphate (0.061 mL, 0.423 mmol). Sodium hydride (60% in mineral oil) (17 mg, 0.423 mmol) was added at 0 °C. The solution was stirred for 1 h at 0 °C and 12 h at room temperature. 20 mL of Amberlite XAD-2 was added to the solution and the beads were thoroughly mixed with the solvent. To the mixture was added ice water 2 mL, and the THF evaporated off. The beads were then washed with distilled water 6 times 100 mL then extracted three times with ethyl acetate (30 mL). The combined phase was evaporated and the residue was dried under high vacuum. The crude product was purified by flash chromatography using ethyl acetate/hexane (8/2), then EtOAc 100% as eluent. The yield of this reaction is 152 mg 61%.
LC-MS: 761.2 (M+H)⁺, 90% pure
¹H NMR (CD₃OD): *δ* 0.68-0.75 (m, 1H), 0.75-0.84 (m, 1H), 0.84-1.10 (m, 9H), 1.21-1.50 (m, 8H), 1.88 (m, 1H), 2.58-2.71 (m, 1H), 2.80-2.89 (m, 1H), 2.89-3.08 (m, 2H), 3.49-3.60 (s, 3H), 3.65-3.74 (m, 1H), 3.85-3.95 (m, 1H), 3.97-4.02 (m, 1H), 4.07-4.21 (m, 4H), 4.29 (d, J = 10.8, 1H), 6.71 (d, J = 8.0, 2H), 7.10-7.20 (m, 2H), 7.20-7.32 (m, 5H), 7.32-7.45 (m, 3H), 7.50 (d, J = 7.5, 2H), 7.86 (br s, 1H).
³¹P NMR (CD₃OD): *δ* 1.62

### Step H. Preparation of (1S,5S)-(1-{5-[(4-amino-benzenesulfonyl)-isobutyl-amino]-6-phosphonooxy-hexylcarbamoyl}-2,2-diphenyl-ethyl)-carbamic acid methyl ester (PL-461)

The product of step G prepared above (152 mg) was dissolved in anhydrous dichloromethane (3.0 mL). Trimethylsilyl bromide (0.5 mL) was added at 0 °C. The mixture was stirred during 1h at this temperature and overnight at room temperature. The solvent was evaporated and 0.2 mL water was added to the residue. 3 mL EtOH was added mixed and evaporated. This step was repeated three times and the residue dried in vacuo. Yields 98 mg 70% of the title derivatives of this first example.
LC-MS: 705.2 (M+H)⁺, 95% pure
¹H NMR (CD₃OD): *δ* 0.65-0.73 (m, 1H), 0.75-0.83 (m, 1H), 0.89 (d, J = 5.6, 8H), 1.27-1.38, (m, 1H), 1.42-4.55 (m, 1H), 1.82-1.94 (m, 1H), 2.57-2.68 (m, 1H), 2.78-2.90 (m, 1H), 2.91-3.09 (m, 2H), 3.54 (s, 3H), 3.60-3.72 (m, 1H), 3.87-4.05 (m, 1H), 4.00 (m, 1H), 4.29 (d, J = 11.3, 1H), 4.90 (d, J = 11.4, 1H), 6.73 (d, J = 8.0, 2H), 7.13-7.22 (m, 2H), 7.22-7.33 (m, 6H), 7.33-7.45 (m, 2H), 7.51 (d, J = 7.5, 2H).
³¹P NMR (CD₃OD): *δ* 2.80

### Example 2. Preparation of (1S,5S)-(1-{5-[(4-amino-benzenesulfonyl)-isobutyl-amino]-6-phosphonooxy-hexylcarbamoyl}-2,2-diphenyl-ethyl)-carbamic acid methyl ester sodium salt (PL-462)

70.7 mg of the final product of example 1 is added to 1 mL 0.1 N NaOH and diluted with 1 mL of distilled water. The Solution is then frozen and lyophilized. Yields 67.2 mg (92%) of the desired material with 95% purity.
¹H NMR (CD₃OD): *δ* 0.72-0.83 (m, 1H), 0.90 (d, J = 5.8, 9H), 1.26-1.38 (m, 1H), 1.53-1.65 (m, 1H), 1.88-2.00 (m, 1H), 2.60-2.70 (m, 1H), 2.79-2.89 (m, 1H), 2.98-3.00 (m, 1H), 3.00-3.08 (m, 1H), 3.54 (s, 3H), 3.58-3.71 (m, 1H), 3.72-3.83 (m, 1H), 3.84-3.95 (m, 1H), 4.28 (d, J = 11.1, 1H), 4.91 (d, J = 11.0, 1H), 6.70 (d, J = 7.6, 2H), 7.12-7.22 (m, 2H), 7.22-7.32 (m, 6H), 7.33-7.40 (m, 2H), 7.50 (d, J = 7.7, 2H).
³¹P NMR (CD₃OD): *δ* 3.13

### Example 3. Preparation of (1S,5S)-(1-{5-[(4-amino-benzenesulfonyl)-isobutyl-amino]-6-phosphonooxy-hexylcarbamoyl}-2-naphthalen-2-yl-ethyl)-carbamic acid methyl ester (PL-507)

The preparation of the title compound is based on scheme 2 of this invention.

### Step A. Preparation of (1S)-(4-{[5-tert-butoxycarbonylamino-1-(diethoxyphosphoryloxymethyl)-pentyl]-isobutyl-sulfamoyl}-phenyl)-carbamic acid tert-butyl ester (VIII)

2.00 g (3.7 mmol) (1*S*)-{4-[(5-tert-butoxycarbonylamino-1-hydroxymethyl-pentyl)-isobutyl-sulfamoyl]-phenyl}-carbamic acid tert-butyl ester (***VII***) (example 1, step D) is dissolved in 0.63 mL triethylphosphate and 10 mL THF at 0 °C under inert argon atmosphere. 0.63 mL (4.44 mmol) diethylchlorophosphate is added and then 0.25 g (6.2 mmol), NaH 60% in oil is added in portionwise. The mixture is allowed to warm to room temperature and left to stir for 2 h (LC-MS showed completion after 1h). To the solution is added 20 mL of Amberlite XAD-2 resin and the slurry thoroughly mixed and added to 200 mL ice water. After stirring for 15 min. the resin suspension is filtered and the resin washed several times with distilled water (500 mL). The desired product is desorbed from the resin with acetone (5 X 50 mL), EtOAc (5 X 50 mL), the organic phase is then dried over Na₂SO₄. After evaporation of the solvent 2.66 g (89%) of clear oil is obtained. The crude product contains a fraction with two diethyl phosphates and is used as is in the next step.
¹H NMR (CD₃OD): *δ* 0.91 (d, J = 6.3, 6H), 1.11-1.21 (m,2H), 1.33 (t, J = 6.9, 10H), 1.43 (s, 9H), 1.53 (s, 10H), 1.90-1.97 (m, 1H), 2.88-2.96 (m, 3H), 2.96-3.04 (m, 1H), 3.81-3.90 (m, 1H), 3.91-3.99 (m, 1H), 4.01-4.14 (m, 4H), 7.61 (d, J = 8.3, 2H), 7.72 (d, J = 8.4, 2H).
³¹P NMR (CD₃OD): *δ* 1.59

### Step B. Preparation of (2S)-phosphoric acid 6-amino-2-[(4-amino-benzenesulfonyl)-isobutyl-amino]-hexyl ester diethyl ester (IX)

The crude product obtained in the previous step (***VIII**,* 2.66 g) is dissolved in 12 mL EtOH. 4 mL of HCl_{conc·} is added and heated briefly to 70 °C then left at room temperature for 3h. The solvent is evacuated and the residue triturated with 50 mL ether. The thick residue is then dissolved in 3 mL ice water and the pH adjusted to 12 with 50% NaOH. The thick slurry obtained is extracted with EtOAc (3 X 50 mL) and the organic phase dried over Na₂SO₄. After filtration of the drying agent the organic phase is evacuated to yield 1.84 g (98%) of the desired product (***IX***).
LC-MS: 480.2 (M+H)⁺, 95% pure.
¹H NMR (CD₃OD): *δ* 0.91 (s, 6H), 1.11-1.26 (m, 3H), 1.28-1.43 (m, 8H), 1.45-1.51 (m, 1H), 1.52-1.61 (m, 1H), 1.89-1.96 (m, 1H), 2.56 (t, J = 6.7, 2H), 2.85-2.91 (m, 1H), 2.98-3.11 (m, 1H), 3.79-3.99 (m, 1H), 3.94 (d, J = 5.3, 1H), 4.09-4.11 (m, 4H), 6.69 (d, J = 7.9, 2H), 7.50 (d, J = 7.9, 2H).
³¹P NMR (CD₃OD): *δ* 1.61

### Step C. Preparation of (2S)-2-methoxycarbonylamino-3-naphthalen-2-yl-propionic acid (or L-Moc-2-naphthylalanine)

To a solution of L-2-naphthylalanine (215 mg, 1 mmol) (Peptech Corp.) in 5 mL 1N NaOH and 0.5 mL saturated Na₂CO₃ (resulting solution at pH 10) was added methoxycarbonyloxysuccinimide (187 mg, 1.1 mmol) dissolved in 5 mL. Afterwards, the reaction mixture was stirred at room temperature for 2 h. The alkaline solution was extracted once with ether (10 mL) and the aqueous phase was acidified with 1N HCl. This was extracted twice with 20 mL EtOAc, and the combined organic phases were washed with 50 mL 1N HCl. The organic phase was dried over Na₂SO₄, filtered and evaporated to an oil, which solidifies to yields 200 mg (73%) of the desired material. This intermediate (referred as the *N*-substituted amino acid) was used without further purification in the next step.

### Step D. Preparation of (1S,5S)-(1-{5-[(4-amino-benzenesulfonyl)-isobutyl-amino]-6-phosphonooxy-hexylcarbamoyl}-2-naphthalen-2-yl-ethyl)-carbamic acid methyl ester (PL-507)

100 mg L-Moc-2-naphthylalanine (step C) was activated with 100 mg EDAC and 57 mg HOBt in 1.5 mL DMF for 30 minutes. Then, 100 mg of phosphoric acid 6-amino-2-[(4-amino-benzenesulfonyl)-isobutyl-amino]-hexyl ester diethyl ester (step B) was added and left to stir at room temperature for 1 h. 40 mL of 1M K₂CO₃ was added to the DMF solution and left for 10 min. 50 mL of EtOAc was then added and the mixture was then agitated vigorously. Separation of the EtOAc phase was effected, followed by extraction with 5% citric acid (50 mL) once, then water (50 mL) 3 times and finally brine. The organic phase was the separated and evaporated. The residue was taken up in 50 mL DCM and re-evaporated. The residue was again taken up in 50 mL DCM and 0.5 mL of TMSBr was added. The solution was left overnight (16 h). The DCM was evacuated and a solution of ice cold MeOH: Water 1:1 was added, stirred briefly and evacuated. The residue was triturated with ether then dissolved in 1N NaOH. The clear solution was extracted with ether and the aqueous phase acidified with 6N HCl. The white precipitated was then collected by filtration and dried in vacuo overnight. Yields 88 mg of the title compound.
LC-MS: 679.8 (M+H)⁺, 95% pure.
¹H NMR (CD₃OD): *δ* 0.89-0.98 (m, 8H), 1.15 (m, 2H), 1.35 (m, 1H), 1.45 (m, 1H), 1.88 (m, 1H), 2.84 (m, 2H), 2.98 (m, 1H), 3.01 (m, 2H), 3.24 (m, 1H), 3.56 (s, 3H), 3.60 (m, 1H), 3.81 (m, 1H), 3.99 (m, 1H), 4.39 (t, J = 6.8, 1H), 6.91 (d, J = 8.0, 2H), 7.34 (d, J= 8.0, 1H), 7.45 (m, 2H), 7.58 (d, J = 8.0, 2H), 7.66 (s, 1H), 7.70-7.82 (m, 3H).
³¹P NMR (CD₃OD): *δ* 2.56

### Example 4. Preparation of (2S,2S) phosphoric acid mono-(2-[(4-amino-benzenesulfonyl)-isobutyl-amino]-6-{2-[(morpholine-4-carbonyl)-amino]-3-naphthalen-1-yl-propionylamino}-hexyl) ester (PL-498)

### Step A. Preparation of (2S)-2-[(morpholine-4-carbonyl)-amino]-3-naphthalen-1-yl-propionic acid

To a solution of L-1-naphthylalanine (215 mg, 1 mmol) (Peptech Corp.) in 5 mL 1N NaOH and 0.5 mL saturated Na₂CO₃ (resulting solution at pH 10) was added morpholine-4-carbonyl chloride (150 mg, 1.0 mmol) dissolved in 5 mL. Afterwards, the reaction mixture was stirred at room temperature for 2 h. The alkaline solution was extracted once with ether (10 mL) and the aqueous phase was acidified with 1N HCl. This was extracted twice with 20 mL EtOAc, and the combined organic phases were washed with 50 mL 1N HCl. The organic phase was dried over Na₂SO₄, filtered and evaporated to an oil, which solidifies to yields 125 mg (38%) of the desired material. This compound was used as such in the next step.

### Step B. Preparation of (2S,2S) Phosphoric acid mono-(2-[(4-amino-benzenesulfonyl)-isobutyl-amino]-6-{2-[(morpholine-4-carbonyl)-amino]-3-naphthalen-1-yl-propionylamino}-hexyl) ester (PL-498)

This compound was made as for the preparation of the product of example 3 (step D) with 100 mg of (2*S*)-2-[(morpholine-4-carbonyl)-amino]-3-naphthalen-1-yl-propionic acid (step A of this example). The resulting precipitated residue was further purified by reverse phase preparative HPLC. Yields 41 mg of the final compound.
LC-MS: 734.8 (M+H)⁺, 95% pure.
¹H NMR (CD₃OD): *δ* 0.83-0.98 (m, 8H), 1.00-1.25 (m, 4H), 1.45-1.52 (m, 1H), 1.52-1.66 (m, 1H), 1.88-1.99 (m, 1H), 2.77-2.92 (m, 2H), 2.98-3.16 (m, 3H), 3.40-3.49 (m, 1H), 3.50-3.56 (m, 6H), 3.67-3.69 (m, 1H), 3.81-3.89 (m, 1H), 3.99-4.05 (m, 1H), 4.59 (t, J = 6.0, 1H), 6.75 (d, J = 8.0, 2H), 7.30-7.60 (m, 7H), 7.75 (d, J = 8.0, 1H), 7.90 (d, J= 7.8, 1H), 8.23 (d, J=7.8 2H).
³¹P NMR (CD₃OD): *δ* 2.71

### Example 5. Preparation of (2S,2S)-phosphoric acid mono-{6-(2-acetylamino-3,3-diphenyl-propionylamino)-2-[(4-amino-benzenesulfonyl)-isobutyl-amino]-hexyl} ester (PL-504)

### Step A. Preparation (2S)-2-acetylamino-3,3-diphenyl-propionic acid

To a solution of L-diphenylalanine (100 mg, 0.4 mmol) (Peptech Corp.) in 5 mL 1N NaOH and 0.5 mL saturated Na₂CO₃ (resulting solution at pH 10) was added acetyl chloride (0.5 mmol) dissolved in 5 mL. Afterwards, the reaction mixture was stirred at room temperature for 2 h. The alkaline solution was extracted once with ether (10 mL) and the aqueous phase was acidified with 1N HCl. This was extracted twice with 20 mL EtOAc, and the combined organic phases were washed with 50 mL 1N HCl. The organic phase was dried over Na₂SO₄, filtered and evaporated to an oil, which solidifies to yields 70 mg (60%) of the desired material. This crude intermediate was used as such in the next step.

### Step B. Preparation of (2S,2S)-phosphoric acid mono-{6-(2-acetylamino-3,3-diphenyl-propionylamino)-2-[(4-amino-benzenesulfonyl)-isobutyl-amino]-hexyl} ester (PL-504)

This compound was made as for the preparation of the product of example 3 (step D) with 100 mg of (2*S*)-2-acetylamino-3,3-diphenyl-propionic acid (this example step A). The final product was obtained in 30% yield (30 mg).
LC-MS: 689.3 (M+H)⁺, 95% pure.
¹H NMR (CD₃OD): *δ* 0.77-1.04 (m, 9H), 1.10-1.17 (m, 1H), 1.23-1.49 (m, 1H), 1.46-1.57 (m, 1H), 1.78 (s, 3H), 1.88-1.99 (m, 1H), 2.80-2.92 (m, 2H), 2.92-3.08 (m, 2H), 3.63-3.75 (m, 1H), 3.79-3.95 (m, 1H), 4.00 (m, 1H), 4.34 (d, J = 11.3, 1H), 5.19-5.28 (m, 1H), 6.77-6.85 (m, 2H), 7.10-7.20 (m, 2H), 7.27-7.33 (m, 6H), 7.32-7.41 (m, 2H), 7.49-7.62 (m, 2H).
³¹P NMR (CD₃OD): *δ* 2.70

### Example 6. Preparation of (1S,5S)-(1-{5-[(4-amino-3-fluoro-benzenesulfonyl)-isobutyl-amino]-6-phosphonooxy-hexylcarbamoyl}-2,2-diphenyl-ethyl)-carbamic acid methyl ester (PL-515)

**First methodology:** The preparation of the title compound is based on scheme 3 of this invention.

### Step A. Preparation of (1-{5-[(4-amino-3-fluoro-benzenesulfonyl)-isobutyl-amino]-6-hydroxy-hexylcarbamoyl}-2,2-diphenyl-ethyl)-carbamic acid methyl ester (X) (PL-337)

The product of example 1, step F (0.624 g, 1 mmol) is dissolved in 5 mL MeCN at 24 °C. SelectFluor 0.35 g (1 mmol) is added in one portion and stirred for 1h. 1 mL of water is added and the solution was injected directly into a preparative reverse-phase HPLC. The product was collected and lyophilized to give 250 mg (38%) yield of a white solid.
LC-MS: 643.3 (M+H)⁺, 99% pure.
¹H NMR (MeOD): *δ* 0.71-0.85 (m 2H), 0.88 (d, J = 6.3, 6H), 0.91-0.96 (m, 2H), 1.21-1.29 (m, 1H), 1.41-1.52 (m, 1H) 1.82-1.92 (m, 1H), 2.61-2.68 (m, 1H), 2.81-2.85 (m, 2H), 2.94-3.05 (m, 2H), 3.38-3.40 (t, J = 5, 1H), 3.49-3.52 (m, 5H), 4.28 (d, J = 10, 1H), 4.87 (d, J = 10, 1H) 6.90 (t, J = 8.3, 1H), 7.20 (m, 2H), 7.28 (m, 3H), 7.33 (m, 3H), 7.39 (m, 4H).

### Step B. Preparation of (1S,5S)-{1-[5-[(4-amino-3-fluoro-benzenesulfonyl)-isobutyl-amino]-6-(diethoxy-phosphoryloxy)-hexylcarbamoyl]-2,2-diphenyl-ethyl}-carbamic acid methyl ester

The product of step A was phosphorylated with chlorodiethylphosphate following the procedure described in example 1, step G. Yields 157 mg, 68%.
LC-MS: 779.3 (M+H)⁺, 95% pure.
¹H NMR (CD₃OD): *δ* 0.82 (m, 1H), 0.92 (d, J = 6.2, 8H), 0.96 (m, 3H), 1.36 (d, J = 3.7, 6H), 1.90 (m, 1H), 2.69 (m, 1H), 2.89 (m, 1H), 2.98 (m, 2H), 3.56 (s, 3H), 3.74 (m, 1H), 3.93 (m, 1H), 4.03 (m, 1H), 4.12 (q, J = 7.5 and 14.8, 4H), 4.32 (d, J = 11.4, 1H), 4.92 (d, J = 11.4, 1H), 6.90 (t, J = 8.3, 1H), 7.20 (m, 2H), 7.28 (m, 3H), 7.33 (m, 3H), 7.39 (m, 4H).
³¹P NMR (CD₃OD): *δ* 1.65

### Step C. Preparation of (1S,5S)-(1-{5-[(4-amino-3-fluoro-benzenesulfonyl)-isobutyl-amino]-6-phosphonooxy-hexylcarbamoyl}-2,2-diphenyl-ethyl)-carbamic acid methyl ester (XI) (PL-515)

Deprotection was effected using the procedure described in example 1, step G. Yields 101 mg.
LC-MS: 723.2 (M+H)⁺, 95% pure.
¹H NMR (CD₃OD): *δ* 0.65-0.77 (m, 1H), 0.77-0.85 (m, 1H), 0.85-1.05 (m, 9H), 1.25-1.39 (m, 1H), 1.40-1.52 (m, 1H), 1.82-1.98 (m, 1H), 2.58-2.72 (m, 1H), 2.82-2.92 (m, 1H), 2.92-3.05 (m, 2H), 3.54 (s, 3H), 3.64-3.75 (m, 1H), 3.80-3.92 (m, 1H), 3.91-4.04 (m, 1H), 4.29 (d, J = 11.4, 1H), 7.19 (t, J = 6.6, 1H), 7.13-7.21 (m, 2H), 7.22-7.33 (m, 6H), 7.34-7.38 (m, 2H), 7.39-7.48 (m, 2H).
³¹P NMR (CD₃OD): *δ* 2.74

**Second methodology:** The preparation of the title compound is based on scheme 4 of this invention.

### Step A. Preparation (1S,5S)-(1-{5-[(4-amino-benzenesulfonyl)-isobutyl-amino]-6-phosphonooxy-hexylcarbamoyl}-2,2-diphenyl-ethyl)-carbamic acid methyl ester (PL-461)

(2*S*)-2-methoxycarbonylamino-3,3-diphenyl-propionic acid ((example 1, step E) 0.9 g, 3 mmol) was activated in DMF (5 mL) with EDAC (1.7 g, 9 mmol) and HOBt (1.2 g, 9 mmol). To the solution was added 1.17 g of (2*S*)-phosphoric acid 6-amino-2-[(4-amino-benzenesulfonyl)-isobutyl-amino]-hexyl ester diethyl ester **(*IX*)** (example 3, step B) and the mixture stirred for 3 h. 20 g of Amberlite XAD-2 resin was then added and the beads were left to soak for 10 min. The resin was transferred into a glass filter and washed thoroughly with distilled water (400 mL) and 200 mL of 1M NaHCO₃. The beads were then washed with 4 X 50 ml portions of MeOH then EtOAc 200 mL. The organic phase was evaporated. The residue was adsorbed onto silica gel and passed through a short silica gel column (EtOAc) to yield 2.4 g (83%) of white solid after evaporation.

NMR identical as in example 1, step H.

### Step B. Preparation (1S,5S)-{1-[5-[(4-amino-3-fluoro-benzenesulfonyl)-isobutyl-amino]-6-(diethoxy-phosphoryloxy)-hexylcarbamoyl]-2,2-diphenyl-ethyl}-carbamic acid methyl ester (XII)

The product of step A above, (1*S*,5*S*)-(1-{5-[(4-amino-benzenesulfonyl)-isobutyl-amino]-6-phosphonooxy hexylcarbamoyl}-2,2-diphenyl-ethyl)-carbamic acid methyl ester (0.555 g, 0.73 mmol) was dissolved in 5 mL MeCN. Selectfluor (0.26 g, 0.7 mmol) was added and the mixture stirred for 30 min. The mixture was purified by reverse phase preparative HPLC and lyophilized to yield 278 mg (48% yield) white solid.
¹H NMR identical as previous entry, see first methodology above.

### Step C. Preparation (1S,5S)-(1-{5-[(4-amino-3-fluoro-benzenesulfonyl)-isobutyl-amino]-6-phosphonooxy-hexylcarbamoyl}-2,2-diphenyl-ethyl)-carbamic acid methyl ester (XIII, in this specific case is compound XI) (PL-515)

The procedure to make this derivative was as described in the deprotection step for the methodology above. Yields 139 mg 70% after reverse phase HPLC.
¹H NMR identical as previous entry, see first methodology above.

### Example 7. Preparation of (2S,2S)-acetic acid 2-[(4-amino-benzenesulfonyl)-isobutyl-amino]-6-(2-methoxycarbonylamino-3,3-diphenyl-propionylamino)-hexyl ester (PL-521)

The preparation of the title derivative is based on scheme 5 of this invention.

### Step A. Preparation of (2S)-acetic acid 6-tert-butoxycarbonylamino-2-[(4-tert-butoxycarbonylamino-benzenesulfonyl)-isobutyl-amino]-hexyl ester (XIV, R_{1A} = CH₃)

To a stirred solution of (1*S*)-{4-[(5-tert-butoxycarbonylamino-1-hydroxymethyl-pentyl)-isobutyl-sulfamoyl]-phenyl}-carbamic acid tert-butyl ester (intermediate product (***VII***) of example 1, step D, 97 mg, 0.18 mmol) in anhydrous CH₂Cl₂ (3 mL) was added *N,N-*dimethylaminopyridine (22 mg, 0.18 mmol) and acetic anhydride (0.014 mL, 0.18 mmol). The mixture was stirred at room temperature for 1 hour. The solvent was evaporated. Ethyl acetate (50 mL) was added and the organic layer was washed with water (30 mL), then dried with Na₂SO₄ and concentrated. The residue was purified by flash chromatography eluting with ethyl acetate. The yield obtained was quantitative (100 mg).
LC-MS: 586.2 (M+H)⁺, 95% pure

### Step B. Preparation of (2S)-acetic acid 6-amino-2-[(4-amino-benzenesulfonyl)-isobutyl-amino]-hexyl ester (XV, R_{1A} = CH₃)

This derivative was prepared from (2*S*)-acetic acid 6-tert-butoxycarbonylamino-2-[(4-tert-butoxycarbonylamino-benzenesulfonyl)-isobutyl-amino]-hexyl ester as described in example 15, step B. The yellow solid (66 mg) was used for the next reaction without purification.
LC-MS: 386.2 (M+H)⁺, 95% pure

### Step C. Preparation of (2S,2S)-acetic acid 2-[(4-amino-benzenesulfonyl-)isobutyl-amino]-6-(2-methoxycarbonylamino-3,3-diphenyl-propionylamino)-hexyl ester (XVI, R_{1A} = CH₃) (PL-521)

This derivative was prepared from (2*S*)-acetic acid 6-amino-2-[(4-amino-benzenesulfonyl)-isobutyl-amino]-hexyl ester (product of step B) as described in example 15, step B. The final product was purified by flash chromatography with a mixture of eluents hexane/ethyl acetate (2/8). A yellow solid was obtained in 70% yield (70 mg).
LC-MS: 667.3 (M+H)⁺, 95% pure
¹H NMR (acetone-d₆): *δ* 0.85-0.97 (m, 12H), 1.21-1.41 (m, 2H), 1.88-2.00 (s, 3H), 2.59-2.69 (m, 1H), 2.83-2.90 (m, 1H), 2.90-3.01 (m, 1H), 3.01-3.10 (br s, 1H), 3.45-3.60 (s, 3H), 3.70-3.80 (m, 1H), 3.93-4.00 (m, 1H), 4.00-4.11 (m, 1H), 4.38-4.45 (d, J = 11.0, 1H), 4.89-4.98 (t, J = 10.0, 1H), 5.43-5.58 (br s, 1H), 6.28-6.48 (d, J = 8.9, 1H), 6.72-6.83 (d, J = 8.0, 2H), 6.85-6.93 (br s, 1H), 7.12-7.22 (t, J = 7.4, 1H), 7.21-7.31 (d, J = 7.0, 4H), 7.31-7.45 (m, 5H), 7.48-7.57 (d, J = 8.0, 2H).

### Example 8. Preparation of (2S,2S)-nicotinic acid 2-[(4-amino-benzenesulfonyl)-isobutyl-amino]-6-(2-methoxycarbonylamino-3,3-diphenyl-propionylamino)-hexyl ester (PL-520)

### Step A. Preparation of (2S)-nicotinic acid 6-tert-butoxycarbonylamino-2-[(4-tert-butoxycarbonylamino-benzenesulfonyl)-isobutyl-amino]-hexyl ester (XIV, R_{1A} = 3-pyridyl)

(1*S*)-{4-[(5-tert-butoxycarbonylamino-1-hydroxymethyl-pentyl)-isobutyl-sulfamoyl]-phenyl}-carbamic acid tert-butyl ester (intermediate product (***VII***) of example 1, step D, 130 mg, 0.24 mmol) was dissolved in anhydrous DMF (1 mL) and treated with 0.066 mL (0.48 mmol) of triethylamine followed by EDC (120 mg, 0.65 mmol), HOBt (88 mg, 0.65 mmol) and nicotinic acid (27 mg, 0.22 mmol). The mixture was stirred overnight at room temperature. The product was extracted with ethyl acetate (40 mL) and water (40 mL). The organic phase was separated and dried with Na₂SO₄, then evaporated to give 200 mg of crude product. This compound was purified by flash chromatography with ethyl acetate as the eluent. A clear oil was obtained in 100% yield (150 mg).
LC-MS: 649.3 (M+H)⁺, 95% pure
¹H NMR (acetone-d₆): *δ* 0.90-1.14 (d, J = 5.9, 6H), 1.31-1.42 (m, 2H), 1.48 (s, 9H), 1.51-1.55 (m, 2H), 1.59 (s, 9H), 1.62-1.69 (m, 1H), 1.72-1.83 (m, 1H), 3.00-3.11 (m, 2H), 3.11-3.17 (m, 1H), 3.19-3.27 (m, 1H), 4.15-4.24 (m, 1H), 4.35-4.44 (t, J = 9.1, 1H), 4.50-4.58 (dd, J = 4.4 and 11.5, 1H), 5.89-5.99 (br s, 1H), 7.53-7.60 (m, 1H), 7.70-7.77 (d, J = 8.2, 2H), 7.80-7.87 (d, J = 8.2, 2H), 8.24-8.31 (d, J = 7.3, 1H), 8.75-8.82 (m, 1H), 8.82-8.88 (m, 1H), 9.12-9.18 (br s, 1H).

### Step B. Preparation of (2S)-nicotinic acid 6-amino-2-[(4-amino-benzenesulfonyl)-isobutyl-amino]-hexyl ester (XV, R_{1A} = 3-pyridyl)

The product of step A, (2*S*)-nicotinic acid 6-tert-butoxycarbonylamino-2-[(4-tert-butoxycarbonylamino-benzenesulfonyl)-isobutyl-amino]-hexyl ester (150 mg, 0.23 mmol), was dissolved in CH₂Cl₂ (5 mL) and trifluoroacetic acid (1 mL) was added. The mixture was stirred during 2 hours at room temperature. The solvent was evaporated and the residue was extracted with ethyl acetate (40 mL) and NaOH 1M (40 mL) (pH = 10). The organic portion was separated, dried with Na₂SO₄ and evaporated. The residue (100 mg) was used for the next reaction without further purification. The yield was quantitative.
LC-MS: 449.2 (M+H)⁺, 95% pure

### Step C. Preparation of (2S,2S)-nicotinic acid 2-[(4-amino-benzenesulfonyl)-isobutyl-amino]-6-(2-methoxycarbonylamino-3,3-diphenyl-propionylamino)-hexyl ester (PL-520)

The product of step B, (2*S*)-nicotinic acid 6-amino-2-[(4-amino-benzenesulfonyl)-isobutyl-amino]-hexyl ester (100 mg, 0.22 mmol) was dissolved in anhydrous DMF (2 mL) and treated with 0.062 mL (0.45 mmol) of triethylamine followed by EDC (100 mg, 0.56 mmol), HOBt (75 mg, 0.56 mmol) and (2*S*)-2-methoxycarbonylamino-3,3-diphenyl-propionic acid (56 mg, 0.19 mmol). The mixture was stirred overnight at room temperature. The product was extracted with ethyl acetate (40 mL) and water (40 mL). The organic layer was separated and dried with Na₂SO₄, then evaporated to give 160 mg of crude oil. The residue was purified by flash chromatography with a mixture of eluents hexane/ethyl acetate (2/8). The title compound was obtained as a clear oil in 20% yield (25 mg).
LC-MS: 730.2 (M+H)⁺, 95% pure
¹H NMR (acetone-d₆): *δ* 0.80-0.97 (m, 9H), 0.97-1.13 (m, 2H), 1.26-1.40 (m, 1H), 1.40-1.57 (m, 1H), 2.61-2.73 (m, 1H), 2.86-2.98 (m, 2H), 3.00-3.17 (m, 2H), 3.45-3.59 (s, 3H), 3.91-4.00 (m, 1H), 4.24-4.34 (m, 1H), 4.34-4.47 (m, 2H), 4.90-4.99 (t, J = 9.7, 1H), 6.35-6.44 (m, 1H), 6.68-6.79 (d, J = 7.9, 1H), 6.91-7.00 (br s, 1H), 7.13-7.22 (m, 2H), 7.22-7.31 (m, 3H), 7.35-7.48 (m, 4H), 7.49-7.64 (m, 2H), 7.75-7.84 (m, 1H), 8.25-8.36 (m, 1H), 8.76-8.88 (br s, 1H), 9.12-9.26 (br s, 1H).

### Example 9. Preparation of (2S,2S)-dimethylamino-acetic acid 2-[(4-amino-benzenesulfonyl)-isobutyl-amino]-6-(2-methoxycarbonylamino-3,3-diphenyl-propionylamino)-hexyl ester (PL-534)

### Step A. Preparation of (2S)-dimethylamino-acetic acid 6-tert-butoxycarbonylamino-2-[(4-tert-butoxycarbonylamino-benzenesulfonyl)-isobutyl-amino]-hexyl ester (XIV, R_{1A} = (CH₃)₂NCH₂-)

This title compound was obtained from (1*S*)-{4-[(5-tert-butoxycarbonylamino-1-hydroxymethyl-pentyl)-isobutyl-sulfamoyl]-phenyl}-carbamic acid tert-butyl ester (intermediate product **(*VII*)** of example 1, step D) as described example 15, step A using *N,N-*dimethylglycine. The clear oil was obtained in 100% yield (150 mg).
LC-MS: 629.3 (M+H)⁺, 95% pure
¹H NMR (acetone-d₆): *δ* 0.81-0.95 (d, J = 6.1, 6H), 1.18-1.30 (m, 2H), 1.32-1.43 (s, 9H), 1.43-1.52 (s, 8H), 1.52-1.62 (m, 1H), 1.93-2.00 (m, 1H), 2.19-2.29 (s, 4H), 2.69-2.80 (m, 4H), 2.90-3.05 (m, 6H), 3.60-3.65 (m, 1H), 3.85-3.97 (m, 1H), 3.98-4.08 (m, 1H), 4.08-4.14 (m, 1H), 5.78-5.88 (m, 1H), 7.68-7.80 (m, 3H), 8.80-8.88 (br s, 1H).

### Step B. Preparation of (2S)-dimethylamino-acetic acid 6-amino-2-[(4-amino-benzenesulfonyl)-isobutyl-amino]-hexyl ester (XV, R_{1A} = (CH₃)₂NCH₂-)

The title derivative was prepared from (2*S*)-dimethylamino-acetic acid 6-tert-butoxycarbonylamino-2-[(4-tert-butoxycarbonylamino-benzenesulfonyl)-isobutyl-amino]-hexyl ester as described in example 15, step B. The final product (100 mg) was used as such in the next step.
LC-MS: 429.3 (M+H)⁺, 90% pure

### Step C. Preparation of (2S,2S)-dimethylamino-acetic acid 2-[(4-amino-benzenesulfonyl)-isobutyl-amino]-6-(2-methoxycarbonylamino-3,3-diphenyl-propionylamino)-hexyl ester (PL-534)

This title compound was prepared from (2*S*)-dimethylamino-acetic acid 6-amino-2-[(4-amino-benzenesulfonyl)-isobutyl-amino]-hexyl ester as described in example 15, step C. The crude product was purified by LC-preparative. The final compound was obtained in 10% yield (10 mg).
LC-MS: 710.3 (M+H)⁺, 92% pure
¹H NMR (acetone-d₆): *δ* 0.81-0.98 (m, 12H), 1.14-1.30 (m, 2H), 1.31-1.45 (m, 1H), 2.58-2.77 (m, 2H), 2.79-2.90 (m, 2H), 3.42-3.56 (s, 3H), 3.75-3.85 (m, 1H), 3.99-4.17 (m, 3H), 4.23-4.35 (m, 1H), 4.36-4.45 (m, 1H), 4.86-4.96 (m, 1H), 6.33-6.42 (m, 1H), 6.74-6.83 (m, 1H), 6.85-6.90 (m, 1H), 7.12-7.22 (m, 3H), 7.23-7.31 (m, 4H), 7.31-7.44 (m, 5H), 7.47-7.55 (m, 1H), 7.73-7.80 (m, 1H).

### Example 10. Preparation of (2S,2S)-2-amino-3-methyl-butyric acid 2-[(4-amino-benzenesulfonyl)-isobutyl-amino]-6-(2-methoxycarbonylamino-3,3-diphenyl-propionylamino)-hexyl ester (PL-530)

### Step A. Preparation of (2S)-2-benzyloxycarbonylamino-3-methyl-butyric acid 6-tert-butoxycarbonylamino-2-[(4-tert-butoxycarbonylamino-benzenesulfonyl)-isobutyl-amino]-hexyl ester (XIV, R_{1A} = (CH₃)₂CHCH(NH₂)-)

This title compound was obtained from (1*S*)-{4-[(5-tert-butoxycarbonylamino-1-hydroxymethyl-penty1l)-isobutyl-sulfamoyl]-phenyl}-carbamic acid tert-butyl ester (intermediate product (***VII***) of example 1, step D) as described in example 15, step A using (2*S*)-2-benzyloxycarbonylamino-3-methyl-butyric acid. The crude product was purified by flash chromatography eluting with a mixture of hexane/ethyl acetate (1/1). The yield obtained was 100% (150 mg).
LC-MS: 777.3 (M+H)⁺, 95% pure
¹H NMR (acetone-d₆): *δ* 0.80-1.00 (m, 14), 1.13-1.28 (s, 2H), 1.30-1.44 (s, 11H), 1.45-1.56 (s, 10), 1.58-1.67 (m, 1H), 2.87-3.04 (m, 4H), 3.84-3.97 (m, 1H), 3.97-4.12 (m, 2H), 4.12-4.21 (m, 1H), 4.99-5.14 (m, 2H), 5.78-5.89 (m, 1H), 6.38-6.52 (m, 1H), 7.24-7.34 (m, 1H), 7.34-7.41 (m, 2H), 7.65-7.83 (m, 4H), 8.77-8.86 (m, 1H).

### Step B. Preparation of (2S)-benzyloxycarbonylamino-3-methyl-butyric acid 6-amino-2-[(4-amino-benzenesulfonyl)-isobutyl-amino]-hexyl ester (XV, R_{1A} = (CH₃)₂CHCH(NH₂)-)

This derivative was prepared from (2*S*)-2-benzyloxycarbonylamino-3-methyl-butyric acid 6-tert-butoxycarbonylamino-2-[(4-tert-butoxycarbonylamino-benzenesulfonyl)-isobutyl-amino]-hexyl ester (product of step A) as described in example 15, step B. The final compound was obtained in quantitative yield (110 mg) and used for the next step without purification.
LC-MS: 577.3 (M+H)⁺, 90% pure

### Step C. Preparation of (2S,2S)-2-benzyloxycarbonylamino-3-methyl-butyric acid 2-[(4-amino-benzenesulfonyl)-isobutyl-amino]-6-(2-methoxycarbonylamino-3,3-diphenyl-propionylamino)-hexyl ester

The title compound was obtained from (2*S*)-benzyloxycarbonylamino-3-methyl-butyric acid 6-amino-2-[(4-amino-benzenesulfonyl)-isobutyl-amino]-hexyl ester (product of step B) as described in example 15, step C. The clear oil was obtained in 86% yield (120 mg).
LC-MS: 858.3 (M+H)⁺, 95% pure

### Step D. Preparation of (2S,2S)-2-amino-3-methyl-butyric acid 2-[(4-amino-benzenesulfonyl)-isobutyl-amino]-6-(2-methoxycarbonylamino-3,3-diphenyl-propionylamino)-hexyl ester (PL-530)

To a stirred solution of (2*S*,2*S*)-2-benzyloxycarbonylamino-3-methyl-butyric acid 2-[(4-amino-benzenesulfonyl)-isobutyl-amino]-6-(2-methoxycarbonylamino-3,3-diphenyl-propionylamino)-hexyl ester (step C, 120 mg, 0.14 mmol) in anhydrous THF (8 mL), under nitrogen atmosphere, was added palladium 10% wt. on activated carbon (160 mg). The mixture was reacted under hydrogen atmosphere overnight, at room temperature. The solution was filtered and the palladium on carbon was washed with THF (50 mL). The solvent was evaporated and the residue (110 mg) was purified by flash chromatography using ethyl acetate as the eluent. The clear oil was obtained in 47% yield (47 mg).
LC-MS: 796.4 (M+H)⁺, 95% pure
¹H NMR (acetone-d₆): *δ* 0.84-0.97 (m, 12H), 0.97-1.08 (m, 2H), 1.27-1.43 (m, 3H), 1.49-1.62 (m, 4H), 1.80-1.93 (m, 1H), 1.94-2.00 (m, 1H), 2.36-2.46 (m, 1H), 2.58-2.74 (m, 2H), 2.86-2.96 (m, 3H), 2.99-3.10 (m, 2H), 3.46-3.52 (s, 3H), 3.52-3.60 (m, 2H), 3.75-3.87 (m, 2H), 3.95-4.04 (m, 1H), 4.10-4.18 (m, 1H), 4.37-4.44 (m, 1H), 4.89-4.97 (m, 1H), 5.40-5.48 (m, 1H), 6.30-6.40 (m, 1H), 6.76-6.83 (d, J = 8.2, 1H), 6.87-7.03 (m, 2H), 7.14-7.22 (m, 1H), 7.23-7.34 (m, 3H), 7.35-7.45 (m, 4H), 7.50-7.56 (m, 1H), 7.57-7.65 (m, 1H).

### Bioavailability of the compounds

To assess the extent of in vivo cleavage of the phosphate group from the putative compounds, PL-100, PL-462 (based on PL-100), PL-337 and PL-515 (based on PL-337) compounds were administered po (50 mg/kg) to male Sprague-Dawley rats and their plasma concentration measured at different time intervals post-administration.

PL100 is an active ingredient (protease inhibitor) of the following formula;

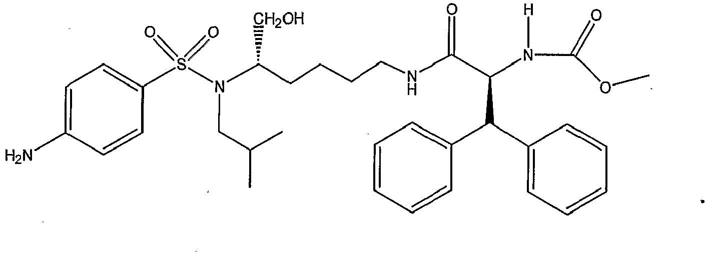

PL-337 is an active ingredient (protease inhibitor) of the following formula;

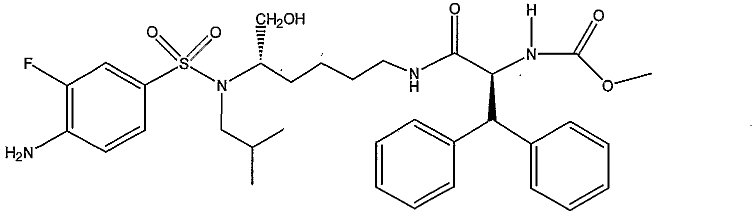

The active ingredient has been shown to be efficient against an HIV-1 aspartyl protease (US patent no. 6,632,816). The active ingredients also present potent antiviral activity when tested on non-mutated HIV-1 viral strain (NL4.3 as the wild type virus) as well as several mutant strains.

All test articles (PL-100, PL-462, PL-337 and PL-515) were prepared in different vehicle at the final concentration of 25 mg/mL. The vehicle composition is as follows: (1) 20% ethanol; 50% propylene glycol; 0.05% w/v Tween 20 and water (Mix); (2) PBS buffer (PBS).

Test articles were administered to male Sprague-Dawley rats at a single oral dose of 50 mg/kg. Each article was tested in three rats. Blood samples (0.2-0.3 mL) were collected at the post-dose time of 10, 20, 40, 60,120, 180 and 360 minutes. The harvested blood was centrifuged to isolate plasma. The resulting plasma was separated and stored at -70°C.

Plasma samples together with standards and quality control samples were treated to precipitate proteins, then analyzed by HPLC-MS, for the presence of PL-462, PL-100, PL-515 and PL-337.

**Table 1**

| Compound | **PL-462** (Ex. No. 2) | **PL-100** (Ex. No. 1-F) | **PL-515** (Ex. No. 13) | **PL-337** (Ex. No. 13-A) |
|---|---|---|---|---|
| Vehicle | PBS | Mix | PBS | Mix |
| Number of rats | 3 | 3 | 3 | 3 |
| Dose (mg/Kg) | 50 po | 50 po | 50 po | 50 po |
| AUC (µg/hr*ml) | 0.816 ± 0.295 (PL-100, detected) | 0.675 ± 0.171 | 1.075 ± 0.625 (PL-337, detected) | 1.180 ± 0.196 |
| Cmax (nM) | 330 ± 109 | 498 ± 203 | 545 ± 215 | 681 ± 131 |
| Tmax (min) | 93 ± 60 | 40 ± 16 | 87 ± 60 | 60 ± 15 |

| | | | | |
|---|---|---|---|---|
| 50 mg/Kg **PL-462** = 43 mg/Kg **PL-100** 50 mg/Kg **PL-515** = 43 mg/Kg **PL-337** | | | | |

The results demonstrate that PL-462 and PL-515 compounds may be delivered orally in aqueous solutions. None of the PL-462 and PL-515 compounds, delivered as aqueous solutions, are detected in the blood samples, which suggests rapid metabolism to PL-100 and PL-337 the parent drugs.

Aqueous dosing of PL-462 and PL-515 solutions showed equivalent to slightly superior delivery of PL-100 and PL-337 compared to non-aqueous formulations of PL-100 and PL-337.

Based on these results, all the phosphorylated compounds described in the present invention will demonstrate similar pharmacokinetic properties.

Partition coefficient (LogP) of selected compounds and the corresponding HIV protease inhibitors (drug) are as follow:

**Table 2.**

| **Compounds** | **LogP** | **Corresponding drugs** | **LogP** |
|---|---|---|---|
| PL-461 (or PL-462) | -1.2 | PL-100 | 3.6 |
| PL-515 | -0.75 | PL-337 | 3.8 |

The LogP were measured in a standard fashion by dissolving 1 mg of compound in 0.8 mL of each octanol and phosphate buffer pH 7.4 (0.04 M KHPO₄). The concentration of the compounds in the phases was detected by LC-MS. This test demonstrates the solubility of the compounds at physiological pH. The LogP obtained show that the compounds are highly soluble as compare to the corresponding drugs.

The compounds listed in Table 3 were prepared by following scheme 1, 1A, 2, 3, 4 or 5; and more particularly as described in each example listed above. The numbers of the compounds listed in Table 3 (Ex. No.) corresponds to the example numbers presented above.

**Table 3: Structures of lysine based compounds in accordance with the present invention**

| | | | | | | | | | |
|---|---|---|---|---|---|---|---|---|---|
| 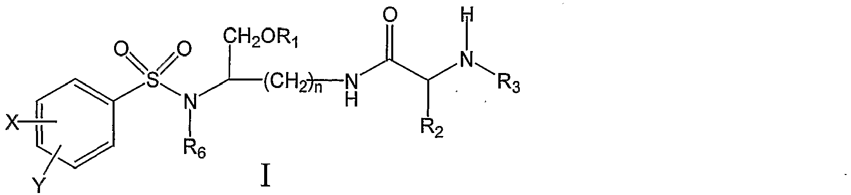 | | | | | | | | | |

| Ex. No (PL-#) | X | Y | n | R₁ | R₂ | R₃ | R₆ | X'/Y' | D, L, DL *R,S,RS* |
|---|---|---|---|---|---|---|---|---|---|
| 1 (PL-461) | 4-NH₂ | H | 4 | (HO)₂P(O) | (C₆H₅)₂CH | CH₃O-CO | *iso*-butyl | H/H | *S,S* |
| 2 (PL-462) | 4-NH₂ | H | 4 | (NaO)₂P(O) | (C₆H₅)₂CH | CH₃O-CO | *iso*-butyl | H/H | *S,S* |
| 3 (PL-507) | 4-NH₂ | H | 4 | (HO)₂P(O) | Naphthyl-2-CH₂ | CH₃O-CO | *iso*-butyl | H/H | *S,S* |
| 4 (PL-498) | 4-NH₂ | H | 4 | (HO)₂P(O) | Naphthyl-1-CH₂ | 4-morpholine-CO | *iso*-butyl | H/H | *S,S* |
| 5 (PL-504) | 4-NH₂ | H | 4 | (HO)₂P(O) | (C₆H₅)₂CH | CH₃CO | *iso*-butyl | H/H | *S,S* |
| 6 (PL-515) | 4-NH₂ | 3-F | 4 | (HO)₂P(O) | (C₆H₅)₂CH | CH₃O-CO | *iso*-butyl | H/H | *S,S* |
| 7 (PL-521) | 4-NH₂ | H | 4 | CH₃CO | (C₆H₅)₂CH | CH₃O-CO | *iso*-butyl | H/H | *S,S* |
| 8 (PL-520) | 4-NH₂ | H | 4 | 3-Pyridyl-CO | (C₆H₅)₂CH | CH₃O-CO | *iso*-butyl | H/H | *S,S* |
| 9 (PL-534) | 4-NH₂ | H | 4 | (CH₃)₂NCH₂CO | (C₆H₅)₂CH | CH₃O-CO | *iso*-butyl | H/H | *S*,*S* |
| 10 (PL-530) | 4-NH₂ | H | 4 | (CH₃)₂CHCH(NH₂)CO | (C₆H₅)₂CH | CH₃O-CO | *iso*-butyl | H/H | *S,S* |

## Claims

1. A pharmaceutical composition comprising an HIV antiviral or anti-retroviral agent that targets attachment to the cell receptor and cell entry, reverse transcription or viral DNA integration into cellular DNA and a compound of Formula II, 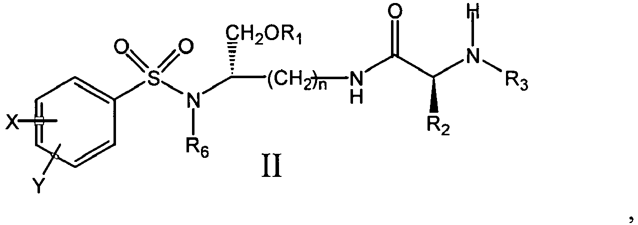 or a pharmaceutically acceptable salt thereof,
wherein n is 3 or 4,
wherein X and Y, the same or different, are selected from the group consisting of H, a straight alkyl group of 1 to 6 carbon atoms, a branched alkyl group of 3 to 6 carbon atoms, a cycloalkyl group of 3 to 6 carbon atoms, F, Cl, Br, I, -CF₃, -OCF₃, -CN, -NO₂, -NR₄R₅,-NHCOR₄, -OR₄, -SR₄, -COOR₄, -COR₄, and -CH₂OH or X and Y together define an alkylenedioxy group selected from the group consisting of a methylenedioxy group of formula -OCH₂O- and an ethylenedioxy group of formula -OCH₂CH₂O-,
wherein R₆ is selected from the group consisting of a straight alkyl group of 1 to 6 carbon atoms, a branched alkyl group of 3 to 6 carbon atoms and a cycloalkylalkyl group having 3 to 6 carbon atoms in the cycloalkyl part thereof and 1 to 3 carbon atoms in the alkyl part thereof,
wherein R₃ is selected from the group consisting of H, a straight alkyl group of 1 to 6 carbon atoms, a branched alkyl group of 3 to 6 carbon atoms, a cycloalkyl group of 3 to 6 carbon atoms, and a group of formula R_{3A}-CO-, R_{3A} being selected from the group consisting of a straight or branched alkyl group of 1 to 6 carbon atoms, a cycloalkyl group having 3 to 6 carbon atoms, a cycloalkylalkyl group having 3 to 6 carbon atoms in the cycloalkyl part thereof and 1 to 3 carbon atoms in the alkyl part thereof, an alkyloxy group of 1 to 6 carbon atoms, tetrahydro-3-furanyloxy, -CH₂OH, -CF₃, -CH₂CF₃, -CH₂CH₂CF₃, pyrrolidinyl, piperidinyl, 4-morpholinyl, CH₃O₂C-, CH₃O₂CCH₂-, Acetyl-OCH₂CH₂-, HO₂CCH₂-, 3-hydroxyphenyl, 4-hydroxyphenyl, 4-CH₃OC₆H₄CH₂-, CH₃NH-, (CH₃)₂N-, (CH₃CH₂)₂N-, (CH₃CH₂CH₂)₂N-, HOCH₂CH₂NH-, CH₃OCH₂O-, CH₃OCH₂CH₂O-, C₆H₅CH₂O-, 2-pyrrolyl, 2-pyridyl, 3-pyridyl, 4-pyridyl-, 2-pyrazinyl, 2-quinolyl, 3-quinolyl, 4-quinolyl, 1-isoquinolyl, 3-isoquinolyl, 2-quinoxalinyl, a phenyl group of formula 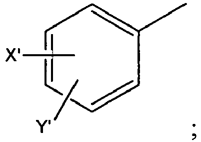 a picolyl group selected from the group consisting of 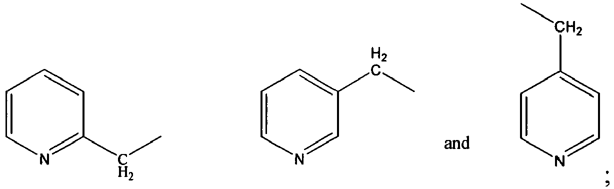 a picolyloxy group selected from the group consisting of 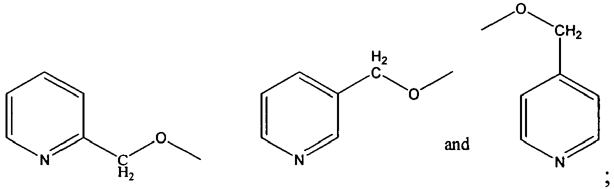 a substituted pyridyl group selected from the group consisting of 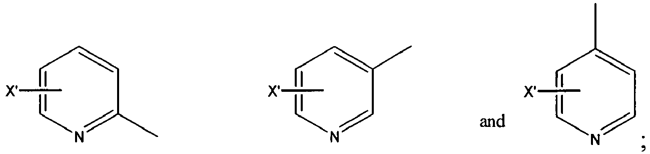 and a group of formula 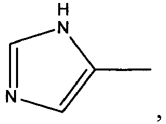 wherein X' and Y', the same or different, are selected from the group consisting of H, a straight alkyl group of 1 to 6 carbon atoms, a branched alkyl group of 3 to 6 carbon atoms, a cycloalkyl group of 3 to 6 carbon atoms, F, Cl, Br, I, -CF₃, -NO₂, -NR₄R₅, -NHCOR₄, -OR₄,-SR₄, -COOR₄, -COR₄ and -CH₂OH,
wherein R₄ and R₅, the same or different, are selected from the group consisting of H, a straight alkyl group of 1 to 6 carbon atoms, a branched alkyl group of 3 to 6 carbon atoms, and a cycloalkyl group of 3 to 6 carbon atoms,
wherein R₂ is selected from the group consisting of a diphenylmethyl group of formula IV, 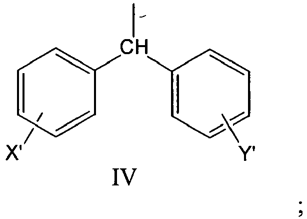 a naphthyl-1-CH₂- group of formula V, 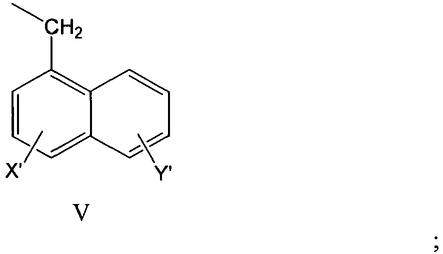 a naphthyl-2-CH₂- group of formula VI, 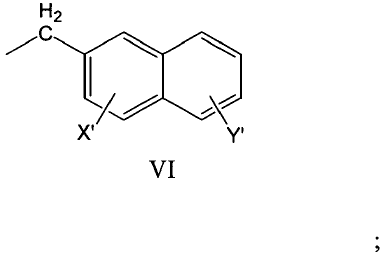 a biphenylmethyl group of formula VII, 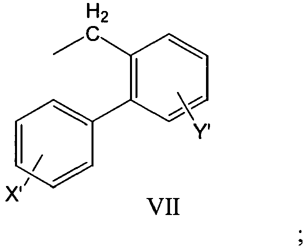 and an anthryl-9-CH₂- group of formula VIII, 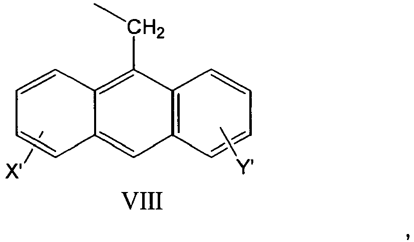 and wherein R₁ is selected from the group consisting of (HO)₂P(O), (MO)₂P(O) and a group of formula R_{1A}-CO-, wherein M is an alkali metal or alkaline earth metal,
wherein R_{1A} is selected from the group consisting of a straight or branched alkyl group of 1 to 6 carbon atoms, a cycloalkyl group having 3 to 6 carbon atoms, a cycloalkylalkyl group having 3 to 6 carbon atoms in the cycloalkyl part thereof and 1 to 3 carbon atoms in the alkyl part thereof, an alkyloxy group of 1 to 6 carbon atoms, -CH₂OH, CH₃O₂C-, CH₃O₂CCH₂-, Acetyl-OCH₂CH₂-, HO₂CCH₂-, 2-hydroxyphenyl, 3-hydroxyphenyl, 4-hydroxyphenyl, (CH₃)₂NCH₂-, (CH₃)₂CHCH(NH₂)-, HOCH₂CH₂NH-, CH₃OCH₂O-, CH₃OCH₂CH₂O-, 2-pyrrolyl, 2-pyridyl, 3-pyridyl, 4-pyridyl, 1-methyl-1,4-dihydro-3-pyridyl, 2-pyrazinyl, 2-quinolyl, 3-quinolyl, 4-quinolyl, 1-isoquinolyl, 3-isoquinolyl, 2-quinoxalinyl, a phenyl group of formula 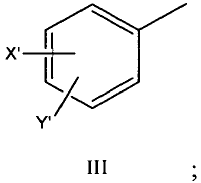 a picolyl group selected from the group consisting of 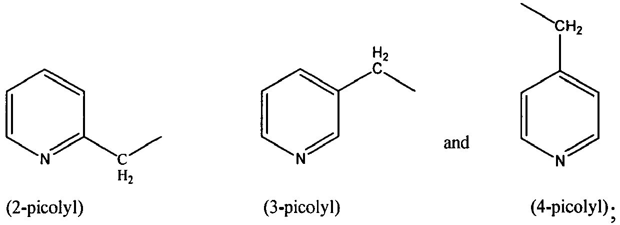 a picolyloxy group selected from the group consisting of 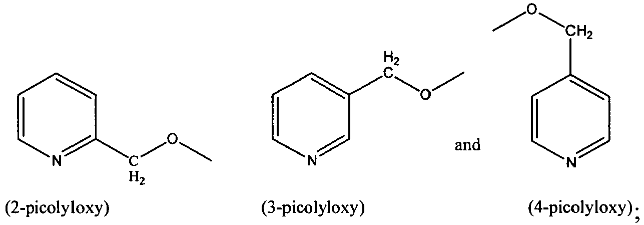 a substituted pyridyl group selected from the group consisting of 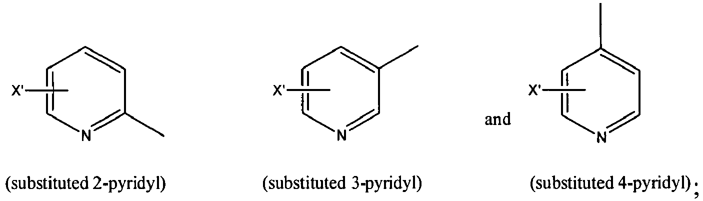 and a group of formula

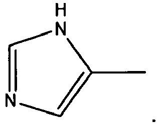

2. A pharmaceutical composition according to claim 1, wherein the compound is of Formula IIa, or of Formula IIb, or of Formula IIc 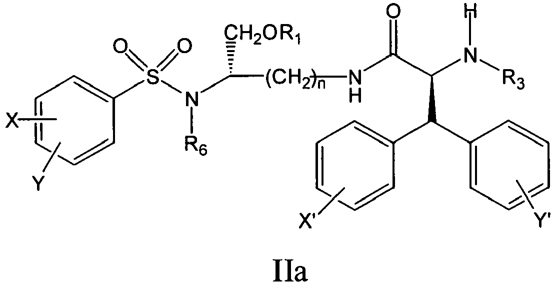 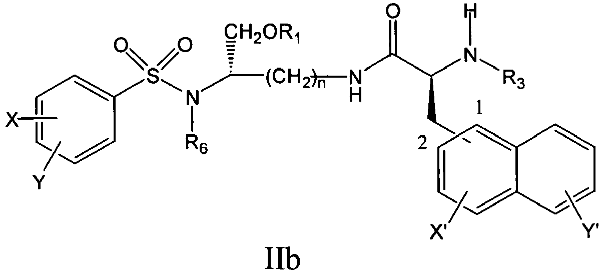 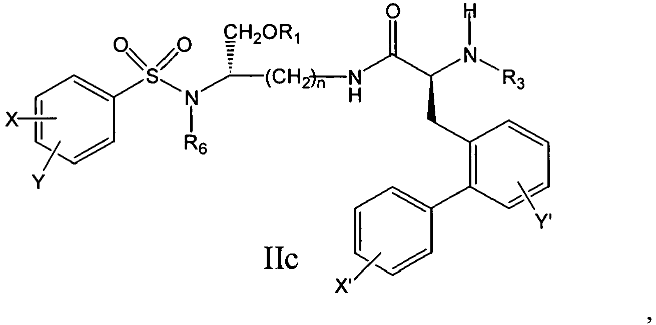 or a pharmaceutically acceptable salt of any of the said compounds.

3. A pharmaceutical composition according to claim 2, wherein R₆ is *iso*-butyl.

4. A pharmaceutical composition according to claim 3, wherein n is 4.

5. A pharmaceutical composition according to claim 4, wherein R₁ is (HO)₂P(O) or (NaO)₂P(O).

6. A pharmaceutical composition according to claim 2, wherein the compound is:
(1*S*,5*S*)-(1-{5-[(4-amino-benzenesulfonyl)-isobutyl-amino]-6-phosphonooxy-hexacarbomoyl}-2,2-diphenyl-ethyl)-carbamic acid methyl ester or a pharmaceutically acceptable salt thereof; or
(1*S*,5*S*)-(1-{5-[(4-amino-3-fluoro-benzenesulfonyl)-isobutyl-amino]-6-phosphonooxy-hexacarbomoyl}-2,2-diphenyl-ethyl)-carbamic acid methyl ester or a pharmaceutically acceptable salt thereof.

7. A pharmaceutical composition according to claim 6, wherein the compound is (1*S*,5*S*)-(1-{5-[(4-amino-benzenesulfonyl)-isobutyl-amino]-6-phosphonooxy-hexacarbomoyl}-2,2-diphenylethyl)-carbamic acid methyl ester sodium salt.

8. A pharmaceutical composition according to claim 6, wherein the compound is (1*S*,5*S*)-(1-{5-[(4-amino-3-fluoro-benzenesulfonyl)-isobutyl-amino]-6-phosphonooxy-hexacarbomoyl}-2,2-diphenyl-ethyl)-carbamic acid methyl ester sodium salt.

9. A pharmaceutical composition according to claim 1, wherein said compound of formula II has a structure according to the following general formula: 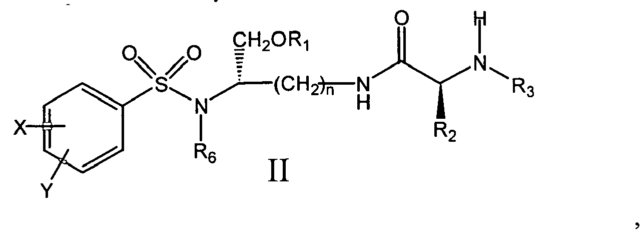 or a pharmaceutically acceptable salt thereof,
wherein n is 4,
wherein X is 4-NH₂ and Y is H or F,
wherein R1 is selected from the group consisting of (HO)₂P(O), (NaO)₂P(O), CH₃CO, 3-pyridyl-CO, (CH₃)₂NCH2CO and (CH₃)₂CHCH(NH₂)CO.
wherein R₂ is selected from the group consisting of a diphenylmethyl group of formula IV, 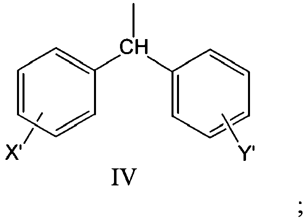 a naphthyl-1-CH₂- group of formula V, 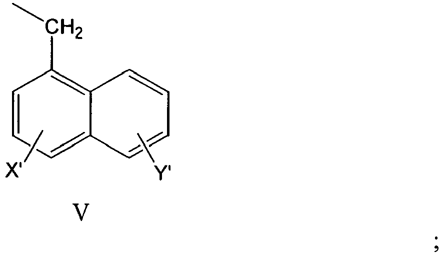 a naphthyl-2-CH₂- group of formula VI, 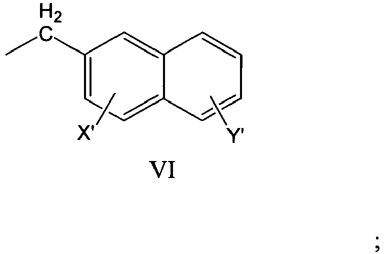 a biphenylmethyl group of formula VII, 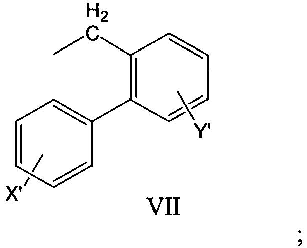 and an anthryl-9-CH₂- group of formula VIII, 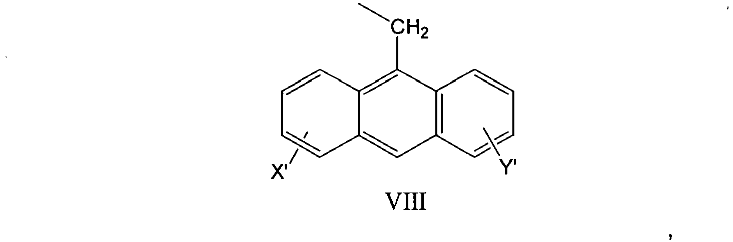 wherein R₃ is selected from the group consisting of CH₃CO, CH₃O-CO, (CH₃)₂N-CO, 3-pyridyl-CO, 4-pyridyl-CO and 4-morpholine-CO
wherein R₄ and R₅, the same or different, are selected from the group consisting of H, a straight alkyl group of 1 to 6 carbon atoms, a branched alkyl group of 3 to 6 carbon atoms, and a cycloalkyl group of 3 to 6 carbon atoms,
and
wherein R₆ is isobutyl.

10. A pharmaceutical composition according to claim 9 wherein R₁ is (HO)₂P(O) or (NaO)₂P(O).

11. A pharmaceutical composition according to claim 9 wherein R₁ is CH₃CO, 3-pyridyl-CO, (CH₃)₂NCH₂CO or (CH₃)₂CHCH(NH₂)CO.

12. A pharmaceutical composition according to any one of claims 1 to 11 further comprising a second compound of Formula II as defined in any one of claims 1 to 11, or a pharmaceutically acceptable salt thereof, or combination thereof, and a pharmaceutically acceptable carrier.

13. The use of at least one pharmaceutical composition as defined in any one of claims 1 to 11 or a combination thereof in the manufacture of a medicament for the treatment or prevention of an HIV infection.

14. The use of a pharmaceutical composition as defined in any one of claims 1 to 11, for the manufacture of a medicament for the treatment or prevention of AIDS-like diseases caused by a retrovirus that depends upon aspartyl proteases similar to those found in HIV for obligatory events in its lifecycle.

15. The use of a pharmaceutical composition as defined in any one of claims 1 to 11 for the manufacture of a medicament for the treatment or prevention of a viral infection selected from the group consisting of simian immunodeficiency virus, HIV-2, HTLV-I and HTLV-II infections.

16. A pharmaceutical composition as defined in any one of claims 1 to 11 for use in the treatment or prevention of AIDS-like diseases caused by a retrovirus that depends upon aspartyl proteases similar to those found in HIV for obligatory events in its lifecycle.

17. A pharmaceutical composition as defined in any one of claims 1 to 11 for use in the treatment or prevention of an HIV infection.

18. A pharmaceutical composition as defined in any one of claims 1 to 11 for use in the treatment or prevention of a viral infection selected from the group consisting of simian immunodeficiency virus, HIV-2, HTLV-I and HTLV-II infections.

19. A pharmaceutical composition according to claim 1, wherein said HIV antiviral is selected from polysulfated polysaccharides, sT4 (soluble CD4) and other compounds which block binding of virus to CD4 receptors on CD4 bearing T-lymphocytes and other CD4(+) cells, or inhibit fusion of the viral envelope with the cytoplasmic membrane, didanosine (ddI), zalcitabine (ddC), stavudine (D4T), zidovudine (AZT), and lamivudine (3TC).

## Patentansprüche

1. Pharmazeutische Zusammensetzung umfassend ein HIV-antivirales oder antiretrovirales Mittel, das auf Anlagern an den Zellrezeptor und Zelleintritt, reverse Transkription oder virale DNA-Integration in zelluläre DNA gerichtet ist, und eine Verbindung der Formel II, 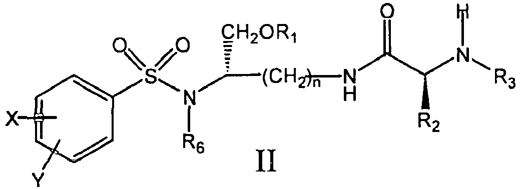 oder ein pharmazeutisch verträgliches Salz davon,
wobei n 3 oder 4 ist,
wobei X und Y, gleich oder verschieden, ausgewählt sind aus der Gruppe bestehend aus H, einer geraden Alkylgruppe von 1 bis 6 Kohlenstoffatomen, einer verzweigten Alkylgruppe von 3 bis 6 Kohlenstoffatomen, einer Cycloalkylgruppe von 3 bis 6 Kohlenstoffatomen, F, Cl, Br, I, -CF₃, -OCF₃, -CN, -NO₂, -NR₄R₅, -NHCOR₄, -OR₄, -SR₄, -COOR₄, -COR₄, und -CH₂OH, oder X und Y zusammen eine Alkylendioxygruppe definieren, ausgewählt aus der Gruppe bestehend aus einer Methylendioxygruppe der Formel -OCH₂O- und einer Ethylendioxygruppe der Formel -OCH₂CH₂O-,
wobei R₆ ausgewählt ist aus der Gruppe bestehend aus einer geraden Alkylgruppe von 1 bis 6 Kohlenstoffatomen, einer verzweigten Alkylgruppe von 3 bis 6 Kohlenstoffatomen und einer Cycloalkylalkylgruppe von 3 bis 6 Kohlenstoffatomen im Cycloalkylteil davon und 1 bis 3 Kohlenstoffatomen im Alkylteil davon,
wobei R₃ ausgewählt ist aus der Gruppe bestehend aus H, einer geraden Alkylgruppe von 1 bis 6 Kohlenstoffatomen, einer verzweigten Alkylgruppe von 3 bis 6 Kohlenstoffatomen, einer Cycloalkylgruppe von 3 bis 6 Kohlenstoffatomen, und einer Gruppe der Formel R_{3A}-CO-, wobei R_{3A} ausgewählt ist aus der Gruppe bestehend aus einer geraden oder verzweigten Alkylgruppe von 1 bis 6 Kohlenstoffatomen, einer Cycloalkylgruppe mit 3 bis 6 Kohlenstoffatomen, einer Cycloalkylalkylgruppe mit 3 bis 6 Kohlenstoffatomen im Cycloalkylteil davon und 1 bis 3 Kohlenstoffatomen im Alkylteil davon, einer Alkoxygruppe von 1 bis 6 Kohlenstoffatomen, Tetrahydro-3-furanyloxy, -CH₂OH, -CF₃, -CH₂CF₃,-CH₂CH₂CF₃, Pyrrolidinyl, Piperidinyl, 4-Morpholinyl, CH₃O₂C-, CH₃O₂CCH₂-, Acetyl-OCH₂CH₂-, HO₂CCH₂-, 3-Hydroxyphenyl, 4-Hydroxyphenyl, 4-CH₃OC₆H₄CH₂-, CH₃NH-, (CH₃)₂N-, (CH₃CH₂)₂N-, (CH₃CH₂CH₂)₂N-, HOCH₂CH₂NH-, CH₃OCH₂O-, CH₃OCH₂CH₂O-, C₆H₅CH₂O-, 2-Pyrrolyl, 2-Pyridyl, 3-Pyridyl, 4-Pyridyl-, 2-Pyrazinyl, 2-Chinolyl, 3-Chinolyl, 4-Chinolyl, 1-Isochinolyl, 3-Isochinolyl, 2-Chinoxalinyl, einer Phenylgruppe der Formel 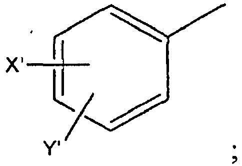 einer Picolylgruppe ausgewählt aus der Gruppe bestehend aus 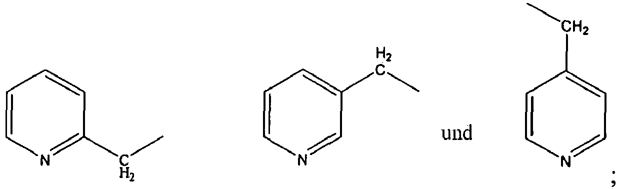 einer Picolyloxygruppe ausgewählt aus der Gruppe bestehend aus 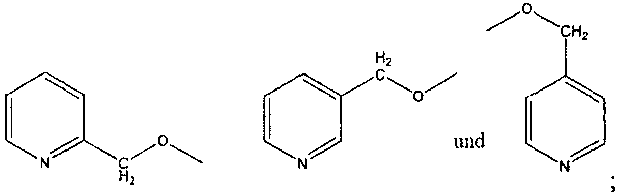 einer substituierten Pyridylgruppe ausgewählt aus der Gruppe bestehend aus 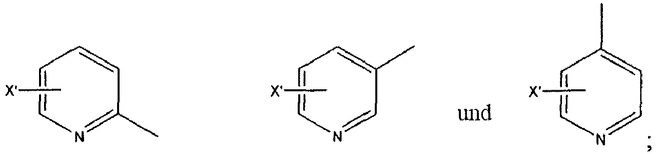 und einer Gruppe der Formel 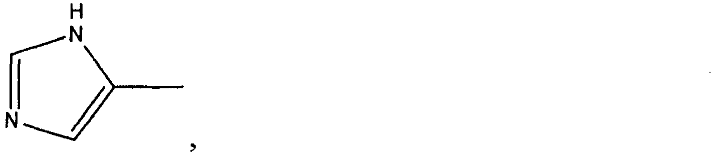 wobei X' und Y', gleich oder verschieden, ausgewählt sind aus der Gruppe bestehend aus H, einer geraden Alkylgruppe von 1 bis 6 Kohlenstoffatomen, einer verzweigten Alkylgruppe von 3 bis 6 Kohlenstoffatomen, einer Cycloalkylgruppe von 3 bis 6 Kohlenstoffatomen, F, Cl, Br, I, -CF₃, -NO₂, -NR₄R₅, -NHCOR₄, -OR₄,-SR₄, -COOR₄, -COR₄, und -CH₂OH,
wobei R₄ und R₅, gleich oder verschieden, ausgewählt sind aus der Gruppe bestehend aus H, einer geraden Alkylgruppe von 1 bis 6 Kohlenstoffatomen, einer verzweigten Alkylgruppe von 3 bis 6 Kohlenstoffatomen, einer Cycloalkylgruppe von 3 bis 6 Kohlenstoffatomen,
wobei R₂ ausgewählt ist aus der Gruppe bestehend aus einer Diphenylmethylgruppe der Formel IV, 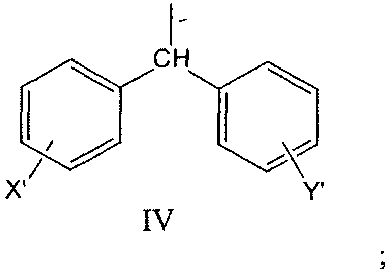 einer Naphthyl-1-CH₂-Gruppe der Formel V, 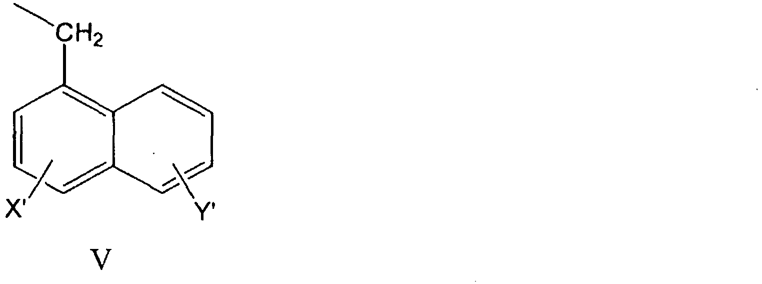 einer Naphthyl-2-CH₂-Gruppe der Formel VI, 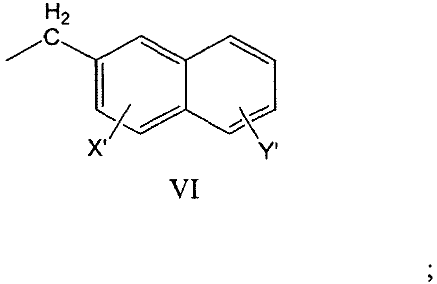 einer Biphenylmethylgruppe der Formel VII, 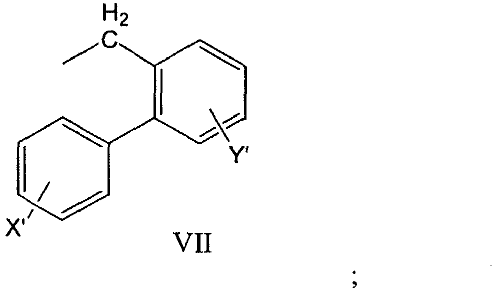 und einer Anthryl-9-CH₂-Gruppe der Formel VIII, 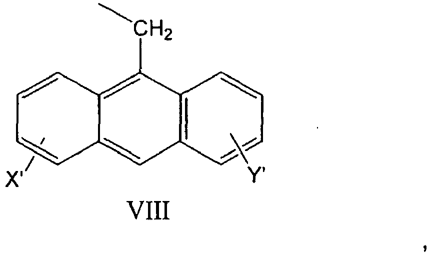 und wobei R₁ ausgewählt ist aus der Gruppe bestehend aus (HO)₂P(O), (MO)₂P(O) und einer Gruppe der Formel R_{1A}-CO-, wobei M ein Alkalimetall oder Erdalkalimetall ist,
wobei R_{1A} ausgewählt ist aus der Gruppe bestehend aus einer geraden oder verzweigten Alkylgruppe von 1 bis 6 Kohlenstoffatomen, einer Cycloalkylgruppe mit 3 bis 6 Kohlenstoffatomen, einer Cycloalkylalkylgruppe mit 3 bis 6 Kohlenstoffatomen im Cycloalkylteil davon und 1 bis 3 Kohlenstoffatomen im Alkylteil davon, einer Alkoxygruppe von 1 bis 6 Kohlenstoffatomen, -CH₂OH, CH₃O₂C-, CH₃O₂CCH₂-, Acetyl-OCH₂CH₂-, HO₂CCH₂-, 2-Hydroxyphenyl, 3-Hydroxyphenyl, 4-Hydroxyphenyl, (CH₃)₂NCH₂-, (CH₃)₂CHCH(NH₂)-, HOCH₂CH₂NH-, CH₃OCH₂O-, CH₃OCH₂CH₂O-, 2-Pyrrolyl, 2-Pyridyl, 3-Pyridyl, 4-Pyridyl, 1-Methyl-1,4-dihydro-3-pyridyl, 2-Pyrazinyl, 2-Chinolyl, 3-Chinolyl, 4-Chinolyl, 1-Isochinolyl, 3-Isochinolyl, 2-Chinoxalinyl, einer Phenylgruppe der Formel 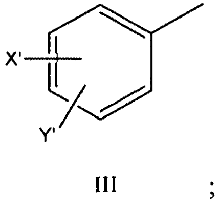 einer Picolylgruppe ausgewählt aus der Gruppe bestehend aus 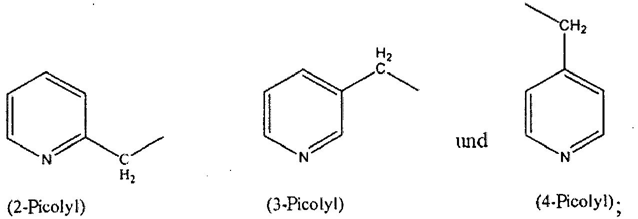 einer Picolyloxygruppe ausgewählt aus der Gruppe bestehend aus 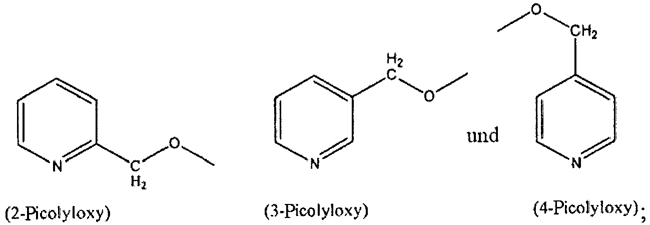 einer substituierten Pyridylgruppe ausgewählt aus der Gruppe bestehend aus 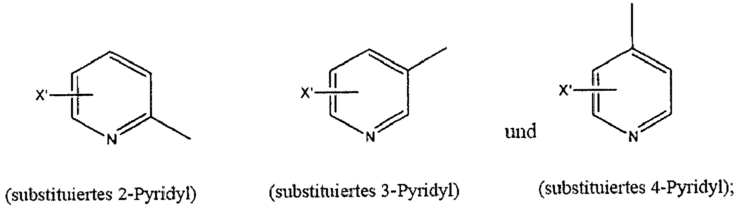 und einer Gruppe der Formel

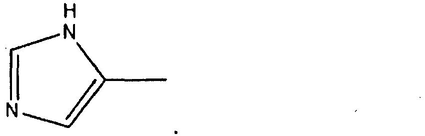

2. Pharmazeutische Zusammensetzung nach Anspruch 1, wobei die Verbindung von der Formel IIa, oder von der Formel IIb oder von der Formel IIc ist 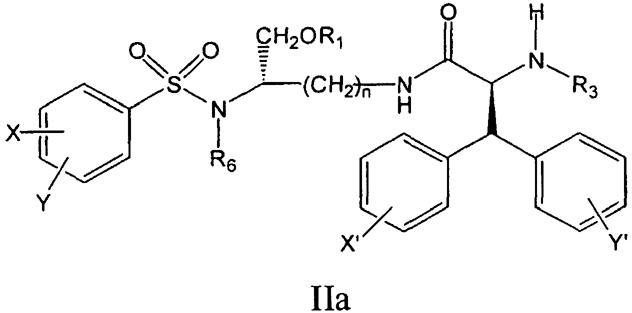 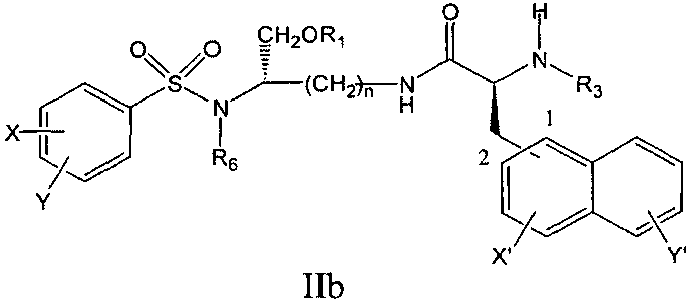 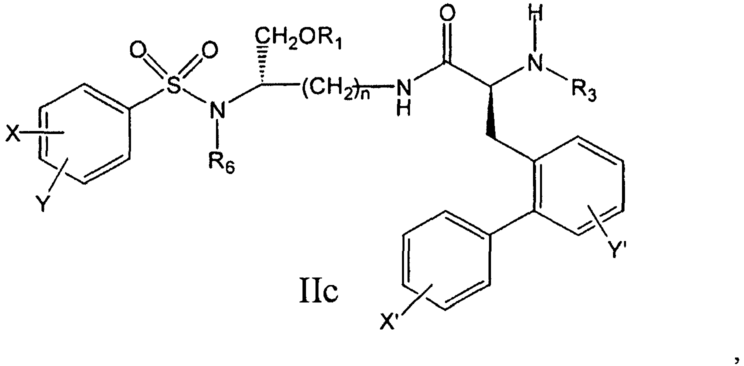 oder ein pharmazeutisch verträgliches Salz einer beliebigen der Verbindungen.

3. Pharmazeutische Zusammensetzung nach Anspruch 2, wobei R₆ *iso*-Butyl ist.

4. Pharmazeutische Zusammensetzung nach Anspruch 3, wobei n 4 ist.

5. Pharmazeutische Zusammensetzung nach Anspruch 4, wobei R₁ (HO)₂P(O) oder (NaO)₂P(O) ist.

6. Pharmazeutische Zusammensetzung nach Anspruch 2, wobei die Verbindung ist:
(1*S*,5*S*)-(1-{5-[(4-Aminobenzolsulfonyl)-isobutylamino]-6-phosphonooxyhexacarbomoyl}-2,2-diphenylethyl)-carbamidsäuremethylester oder ein pharmazeutisch verträgliches Salz davon; oder
(1*S*,5*S*)-(1-{5-[(4-Amino-3-fluorbenzolsulfonyl)-isobutylamino]-6-phosphonooxyhexacarbomoyl}-2,2-diphenylethyl)-carbamidsäuremethylester oder ein pharmazeutisch verträgliches Salz davon.

7. Pharmazeutische Zusammensetzung nach Anspruch 6, wobei die Verbindung (1*S*,5*S*)-(1-{5-[(4-Aminobenzolsulfonyl)-isobutylamino]-6-phosphonooxyhexacarbomoyl}-2,2-diphenylethyl)-carbamidsäuremethylester-Natriumsalz ist.

8. Pharmazeutische Zusammensetzung nach Anspruch 6, wobei die Verbindung (1*S*,5*S*)-(1-{5-[(4-Amino-3-fluorbenzolsulfonyl)-isobutylamino]-6-phosphonooxyhexacarbomoyl}-2,2-diphenylethyl)-carbamidsäuremethylester-Natriumsalz ist.

9. Pharmazeutische Zusammensetzung nach Anspruch 1, wobei die Verbindung der Formel II einer Struktur gemäß der folgenden allgemeinen Formel aufweist: 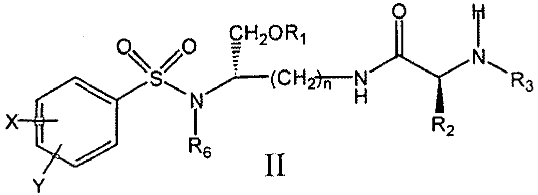 oder ein pharmazeutisch verträgliches Salz davon,
wobei n 4 ist,
wobei X 4-NH₂ ist und Y H oder F ist,
wobei R₁ ausgewählt ist aus der Gruppe bestehend aus (HO)₂P(O), (NaO)₂P(O), CH₃CO, 3-Pyridyl-CO, (CH₃)₂NCH2CO und (CH₃)₂CHCH(NH₂)CO,
wobei R₂ ausgewählt ist aus der Gruppe bestehend aus einer Diphenylmethylgruppe der Formel IV 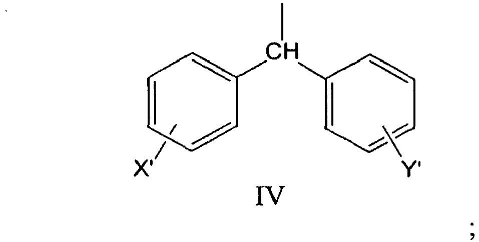 einer Naphthyl-1-CH₂-Gruppe der Formel V, 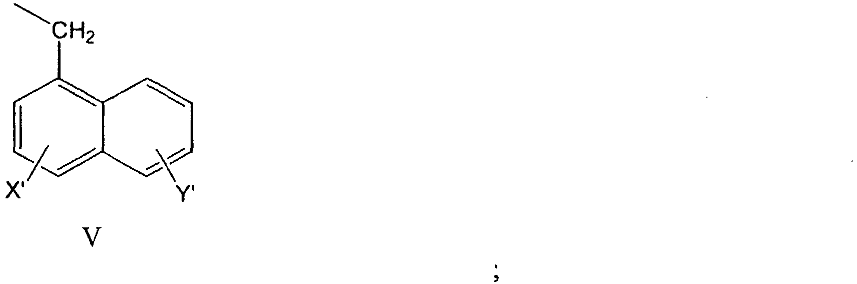 einer Naphthyl-₂-CH₂-Gruppe der Formel VI, 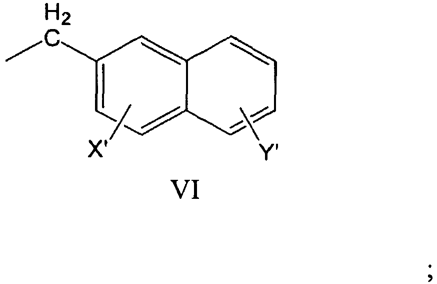 einer Biphenylmethylgruppe der Formel VII, 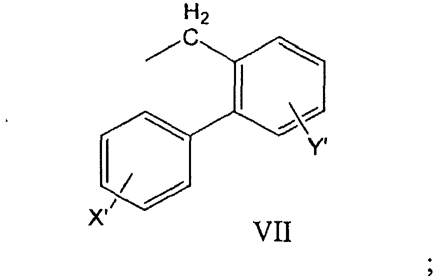 und einer Anthryl-9-CH₂-Gruppe der Formel VIII, 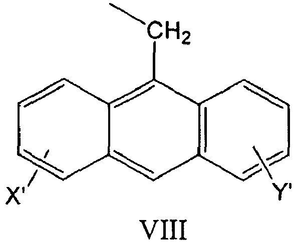 wobei R₃ ausgewählt ist aus der Gruppe bestehend aus CH₃CO, CH₃O-CO, (CH₃)₂N-CO, 3-Pyridyl-CO, 4-Pyridyl-CO und 4-Morpholin-CO
wobei R₄ und R₅, gleich oder verschieden, ausgewählt sind aus der Gruppe bestehend aus H, einer geraden Alkylgruppe von 1 bis 6 Kohlenstoffatomen, einer verzweigten Alkylgruppe von 3 bis 6 Kohlenstoffatomen, einer Cycloalkylgruppe von 3 bis 6 Kohlenstoffatomen,
und
wobei R₆ Isobutyl ist.

10. Pharmazeutische Zusammensetzung nach Anspruch 9, wobei R₁ (HO)₂P(O) oder (NaO)₂P(O) ist.

11. Pharmazeutische Zusammensetzung nach Anspruch 9, wobei R₁ CH₃CO, 3-Pyridyl-CO, (CH₃)₂NCH₂CO oder (CH₃)₂CHCH(NH₂)CO ist.

12. Pharmazeutische Zusammensetzung nach einem beliebigen der Ansprüche 1 bis 11, weiterhin umfassend eine zweite Verbindung der Formel II wie in einem beliebigen der Ansprüche 1 bis 11 definiert, oder ein pharmazeutisch verträgliches Salz davon, oder eine Kombination davon, und ein pharmazeutisch verträglicher Träger.

13. Verwendung mindestens einer pharmazeutischen Zusammensetzung wie in einem beliebigen der Ansprüche 1 bis 11 definiert oder eine Kombination davon in der Herstellung eines Medikaments zur Behandlung oder zur Prävention einer HIV-Infektion.

14. Verwendung einer pharmazeutischen Zusammensetzung wie in einem beliebigen der Ansprüche 1 bis 11 definiert zur Herstellung eines Medikaments zur Behandlung oder zur Prävention von AIDS-artigen Krankheiten, die durch ein Retrovirus verursacht werden, das von Aspartylproteasen ähnlich zu den in HIV gefundenen für obligatorische Vorgänge in seinem Lebenszyklus abhängt.

15. Verwendung einer pharmazeutischen Zusammensetzung wie in einem beliebigen der Ansprüche 1 bis 11 definiert zur Herstellung eines Medikaments zur Behandlung oder zur Prävention einer viralen Infektion ausgewählt aus der Gruppe bestehend aus Simianen Immundefizienz-Virus-, HIV-2-, HTLV-I- und HTLV-II-Infektionen.

16. Pharmazeutische Zusammensetzung wie in einem beliebigen der Ansprüche 1 bis 11 definiert zur Verwendung in der Behandlung oder Prävention von AIDS-artigen Krankheiten, die durch ein Retrovirus verursacht werden, das von Aspartylproteasen ähnlich zu den in HIV gefundenen für obligatorische Vorgänge in seinem Lebenszyklus abhängt.

17. Pharmazeutische Zusammensetzung wie in einem beliebigen der Ansprüche 1 bis 11 definiert zur Verwendung in der Behandlung oder Prävention einer HIV-Infektion.

18. Pharmazeutische Zusammensetzung wie in einem beliebigen der Ansprüche 1 bis 11 definiert zur Verwendung in der Behandlung oder Prävention einer viralen Infektion ausgewählt aus der Gruppe bestehend aus Simianen Immundefizienz-Virus-, HIV-2-, HTLV-I- und HTLV-II-Infektionen.

19. Pharmazeutische Zusammensetzung nach Anspruch 1, wobei das HIV-antivirale Mittel ausgewählt ist aus polysulfatierten Polysacchariden, sT4 (löslichem CD4) und anderen Verbindungen, die Binden von Virus an CD4-Rezeptoren auf CD4-tragenden T-Lymphozyten und anderen CD4(+)-Zellen blockieren, oder Fusion der Virenhülle mit der Zytoplasmamembran inhibieren, Didanosin (ddI), Zalcitabin (ddC), Stavudin (D4T), Zidovudin (AZT) und Lamivudin (3TC).

## Revendications

1. Composition pharmaceutique comprenant un agent antiviral ou anti-rétroviral contre le VIH qui cible la fixation au récepteur cellulaire et la pénétration cellulaire, la transcription inverse ou l'intégration de l'ADN viral dans l'ADN cellulaire et un composé de formule II, 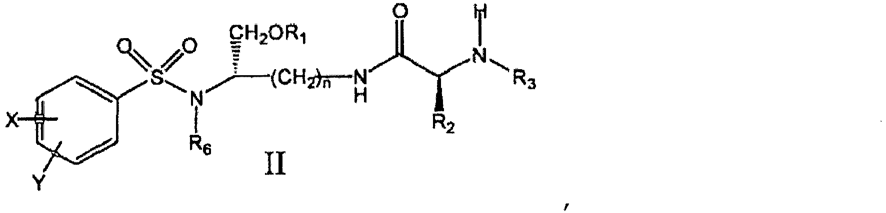 ou un sel pharmaceutiquement acceptable de celui-ci,
formule dans laquelle n vaut 3 ou 4,
dans laquelle X et Y, identiques ou différents, sont choisis dans le groupe constitué de l'atome d'H, d'un groupe alkyle linéaire de 1 à 6 atomes de carbone, d'un groupe alkyle ramifié de 3 à 6 atomes de carbone, d'un groupe cycloalkyle de 3 à 6 atomes de carbone, des atomes de F, CI, Br, I, et des groupes-CF₃, -OCF₃, -CN, -NO₂,-NR₄R₅, - NHCOR₄, -OR₄, -SR₄, -COOR₄, -COR₄ et -CH₂OH ou X et Y définissent ensemble un groupe alkylènedioxy choisi dans le groupe constitué d'un groupe méthylènedioxy de formule -OCH₂O- et d'un groupe éthylènedioxy de formule - OCH₂CH₂O-,
dans laquelle R₆ est choisi dans le groupe constitué d'un groupe alkyle linéaire de 1 à 6 atomes de carbone, d'un groupe alkyle ramifié de 3 à 6 atomes de carbone et d'un groupe cycloalkylalkyle comportant de 3 à 6 atomes de carbone dans la partie cycloalkyle de celui-ci et de 1 à 3 atomes de carbone dans la partie alkyle de celui-ci,
dans laquelle R₃ est choisi dans le groupe constitué de l'atome d'H, d'un groupe alkyle linéaire de 1 à 6 atomes de carbone, d'un groupe alkyle ramifié de 3 à 6 atomes de carbone, d'un groupe cycloalkyle de 3 à 6 atomes de carbone, et d'un groupe de formule R_{3A}-CO-, R_{3A} étant choisi dans le groupe constitué d'un groupe alkyle linéaire ou ramifié de 1 à 6 atomes de carbone, d'un groupe cycloalkyle comportant de 3 à 6 atomes de carbone, d'un groupe cycloalkylalkyle comportant de 3 à 6 atomes de carbone dans la partie cycloalkyle de celui-ci et de 1 à 3 atomes de carbone dans la partie alkyle de celui-ci, d'un groupe alkyloxy de 1 à 6 atomes de carbone, d'un groupe tétrahydro-3-furanyloxy, -CH₂OH, -CF₃, -CH₂CF₃,-CH₂CH₂CF₃, pyrrolidinyle, pipéridinyle, 4-morpholinyle, CH₃O₂C-, CH₃O₂CCH₂-, acétyl-OCH₂CH₂-, HO₂CCH₂-, 3-hydroxyphényle, 4-hydroxyphényle, 4-CH₃OC₆H₄CH₂-, CH₃NH-, (CH₃)₂N-, (CH₃CH₂)₂N-, (CH₃CH₂CH₂)₂N-, HOCH₂CH₂NH-, CH₃OCH₂O-, CH₃OCH₂CH₂O-, C₆H₅CH₂O-, 2-pyrrolyle, 2-pyridyle, 3-pyridyle, 4-pyridyl-, 2-pyrazinyle, 2-quinolyle, 3-quinolyle, 4-quinolyle, 1-isoquinolyle, 3-isoquinolyle, 2-quinoxalinyle, d'un groupe phényle de formule 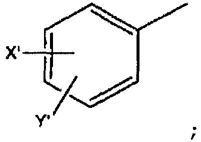 d'un groupe picolyle choisi dans le groupe constitué de 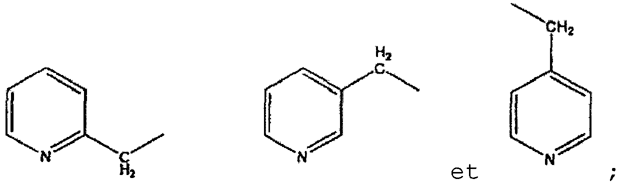 d'un groupe picolyloxy choisi dans le groupe constitué de 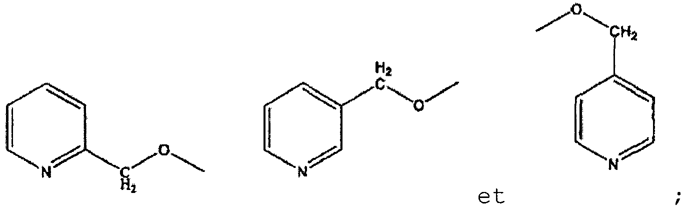 d'un groupe pyridyle substitué choisi dans le groupe constitué de 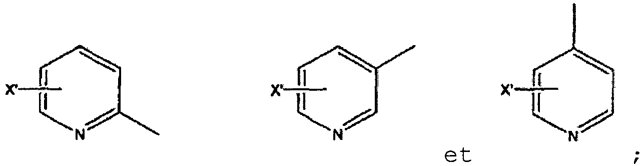 et d'un groupe de formule 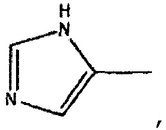 formules dans lesquelles X' et Y', identiques ou différents, sont choisis dans le groupe constitué de l'atome d'H, d'un groupe alkyle linéaire de 1 à 6 atomes de carbone, d'un groupe alkyle ramifié de 3 à 6 atomes de carbone, d'un groupe cycloalkyle de 3 à 6 atomes de carbone, d'un atome de F, Cl, Br, I, et des groupes -CF₃, -NO₂, -NR₄R₅, -NHCOR₄, -OR₄, -SR₄, -COOR₄, -COR₄ et -CH₂OH,
formules dans lesquelles R₄ et R₅, identiques ou différents, sont choisis dans le groupe constitué de l'atome d'H, d'un groupe alkyle linéaire de 1 à 6 atomes de carbone, d'un groupe alkyle ramifié de 3 à 6 atomes de carbone, et d'un groupe cycloalkyle de 3 à 6 atomes de carbone,
dans laquelle R₂ est choisi dans le groupe constitué d'un groupe diphénylméthyle de formule IV, 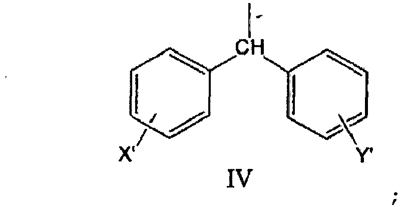 d'un groupe naphtyl-1-CH₂- de formule V, 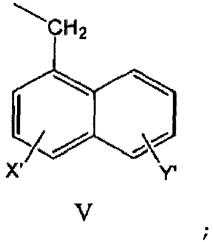 d'un groupe naphtyl-2-CH₂- de formule VI, 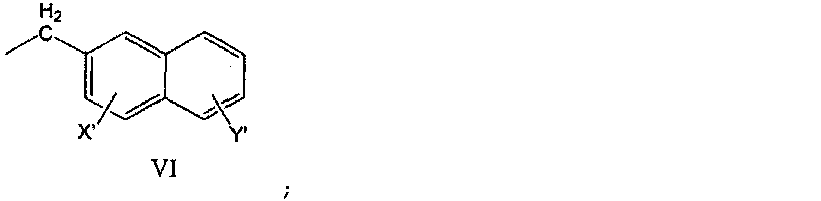 d'un groupe biphénylméthyle de formule VII, 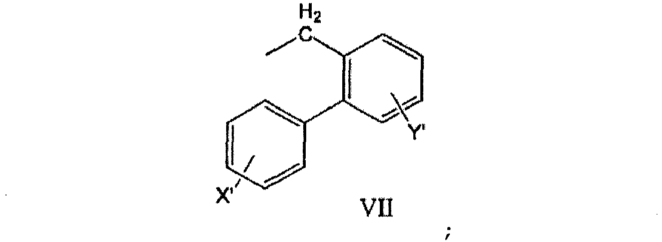 et d'un groupe anthryl-9-CH₂- de formule VIII, 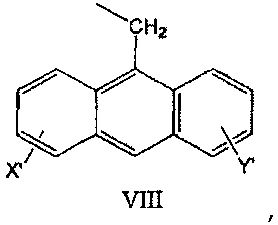 et dans laquelle R₁ est choisi dans le groupe constitué de (HO)₂P(O), (MO)₂P(O) et d'un groupe de formule R_{1A}-CO-, M étant un métal alcalin ou un métal alcalino-terreux, dans laquelle R_{1A} est choisi dans le groupe constitué d'un groupe alkyle linéaire ou ramifié de 1 à 6 atomes de carbone, d'un groupe cycloalkyle comportant de 3 à 6 atomes de carbone, d'un groupe cycloalkylalkyle comportant de 3 à 6 atomes de carbone dans la partie cycloalkyle de celui-ci et de 1 à 3 atomes de carbone dans la partie alkyle de celui-ci, d'un groupe alkyloxy de 1 à 6 atomes de carbone, -CH₂OH, CH₃O₂C-, CH₃O₂CCH₂-, acétyl-OCH₂CH₂-, HO₂CCH₂-, 2-hydroxyphényle, 3-hydroxyphényle, 4-hydroxyphényle, (CH₃)₂NCH₂-, (CH₃)₂CHCH(NH₂)-, HOCH₂CH₂NH-, CH₃OCH₂O-, CH₃OCH₂CH₂O-, 2-pyrrolyle, 2-pyridyle, 3-pyridyle, 4-pyridyle, 1-méthyl-1,4-dihydro-3-pyridyle, 2-pyrazinyle, 2-quinolyle, 3-quinolyle, 4-quinolyle, 1-isoquinolyle, 3-isoquinolyle, 2-quinoxalinyle, d'un groupe phényle de formule 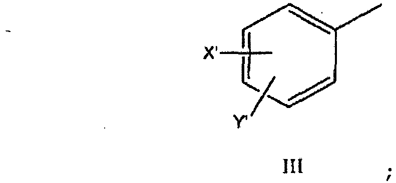 d'un groupe picolyle choisi dans le groupe constitué de 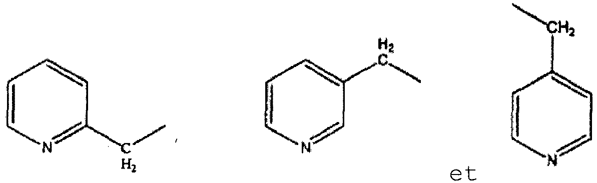 (2-picolyle) (3-picolyle) (4-picolyle) ; d'un groupe picolyloxy choisi dans le groupe constitué de 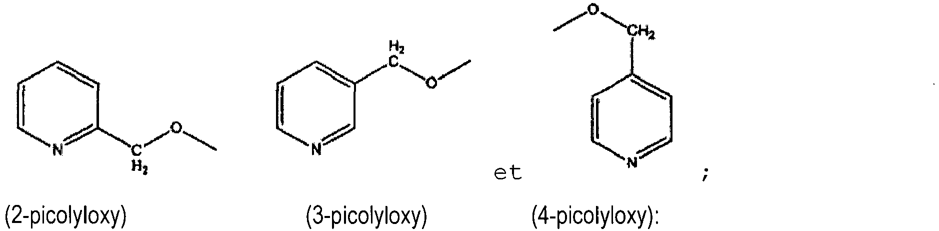 d'un groupe pyridyle substitué choisi dans le groupe constitué de 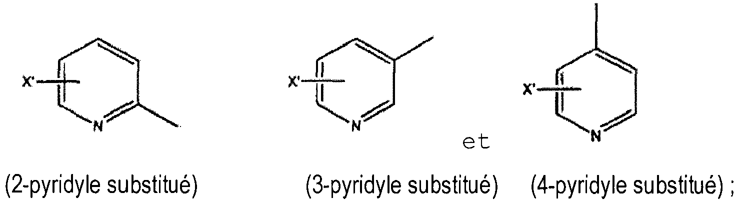 et d'un groupe de formule

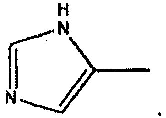

2. Composition pharmaceutique selon la revendication 1, dans laquelle le composé est de Formule IIa, ou Formule IIb, ou Formule IIc 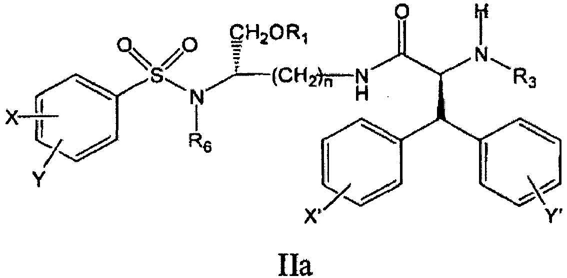 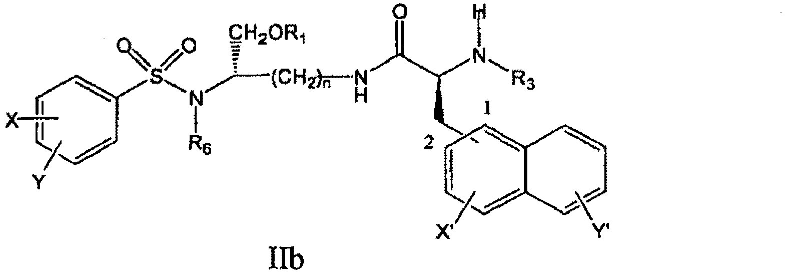 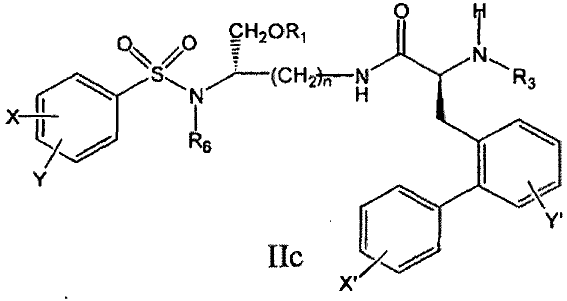 ou un sel pharmaceutiquement acceptable de l'un quelconque desdits composés.

3. Composition pharmaceutique selon la revendication 2, dans laquelle R₆ est de l'*iso*-butyle.

4. Composition pharmaceutique selon la revendication 3, dans laquelle n vaut 4.

5. Composition pharmaceutique selon la revendication 4, dans laquelle R₁ est du (HO)₂P(O) ou du (NaO)₂P(O).

6. Composition pharmaceutique selon la revendication 2, dans laquelle le composé est :
de l'ester méthylique d'acide (1*S*,5*S*)-(1-{5-[(4-amino-benzènesulfonyl)-isobutyl-amino]-6-phosphonooxy-hexacarbomoyl}-2,2-diphényl-éthyl)-carbamique ou un sel pharmaceutiquement acceptable de celui-ci ; ou
de l'ester méthylique d'acide (1*S*,5*S*)-(1-{5-[(4-amino-3-fluoro-benzènesulfonyl)-isobutyl-amino]-6-phosphonooxy-hexacarbomoyl}-2,2-diphényl-éthyl)-carbamique ou un sel pharmaceutiquement acceptable de celui-ci.

7. Composition pharmaceutique selon la revendication 6, dans laquelle le composé est un sel de sodium d'ester méthylique d'acide (1*S*,5*S*)-(1-{5-[(4-amino-benzènesulfonyl)-isobutyl-amino]-6-phosphonooxy-hexacarbomoyl}-2,2-diphényl-éthyl)-carbamique.

8. Composition pharmaceutique selon la revendication 6, dans laquelle le composé est un sel de sodium d'ester méthylique d'acide (1*S*,5*S*)-(1-{5-[(4-amino-3-fluoro-benzènesulfonyl)-isobutyl-amino]-6-phosphonooxy-hexacarbomoyl}-2,2-diphényl-éthyl)-carbamique.

9. Composition pharmaceutique selon la revendication 1, dans laquelle ledit composé de formule II a une structure répondant à la formule générale suivante : 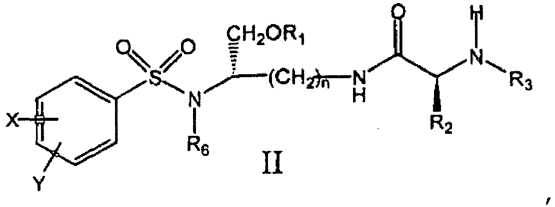 ou un sel pharmaceutiquement acceptable de celui-ci,
formule dans laquelle n vaut 4,
dans laquelle X représente un groupe 4-NH₂ et Y représente un atome d'H ou de F,
dans laquelle R1 est choisi dans le groupe constitué de (HO)₂P(O), (NaO)₂P(O), CH₃CO, 3-pyridyl-CO, (CH₃)₂NCH2CO et (CH₃)₂CHCH(NH₂)CO.
dans laquelle R₂ est choisi dans le groupe constitué d'un groupe diphénylméthyle de formule IV, 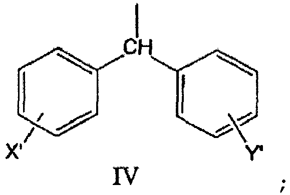 d'un groupe naphtyl-1-CH₂- de formule V, 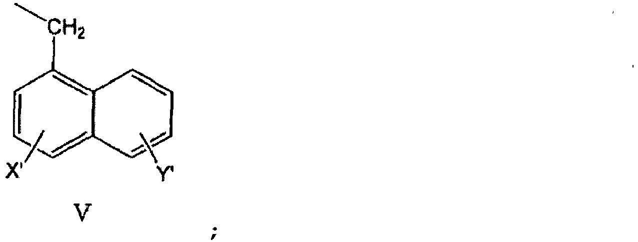 d'un groupe naphtyl-2-CH₂- de formule VI, 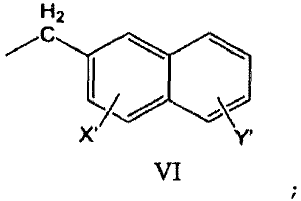 d'un groupe biphénylméthyle de formule VII, 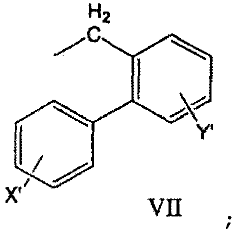 et d'un groupe anthryl-9-CH₂- de formule VIII, 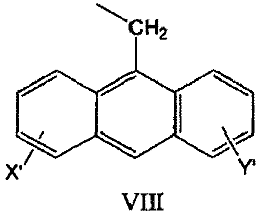 dans laquelle R₃ est choisi dans le groupe constitué de CH₃CO, CH₃O-CO, (CH₃)₂N-CO, 3-pyridyl-CO, 4-pyridyl-CO et 4-morpholine-CO
dans laquelle R₄ et R₅, identiques ou différents, sont choisis dans le groupe constitué de l'atome d'H, d'un groupe alkyle linéaire de 1 à 6 atomes de carbone, d'un groupe alkyle ramifié de 3 à 6 atomes de carbone, et d'un groupe cycloalkyle de 3 à 6 atomes de carbone,
et
dans laquelle R₆ représente un groupe isobutyle.

10. Composition pharmaceutique selon la revendication 9 dans laquelle R₁ est un groupe (HO)₂P(O) ou (NaO)₂P(O).

11. Composition pharmaceutique selon la revendication 9 dans laquelle R₁ est un groupe CH₃CO, 3-pyridyl-CO, (CH₃)₂NCH₂CO ou (CH₃)₂CHCH(NH₂)CO.

12. Composition pharmaceutique selon l'une quelconque des revendications 1 à 11 comprenant en outre un second composé de formule II tel que défini dans l'une quelconque des revendications 1 à 11, ou un sel pharmaceutiquement acceptable de celui-ci, ou une combinaison de ceux-ci, et un vecteur pharmaceutiquement acceptable.

13. Utilisation de l'au moins une composition pharmaceutique telle que définie dans l'une quelconque des revendications 1 à 11 ou d'une combinaison de celles-ci dans la fabrication d'un médicament destiné au traitement ou à la prévention d'une infection par le VIH.

14. Utilisation d'une composition pharmaceutique telle que définie dans l'une quelconque des revendications 1 à 11, pour la fabrication d'un médicament destiné au traitement ou à la prévention de maladies de type SIDA causées par un rétrovirus qui dépend des aspartyl-protéases similaires à celles présentes dans le VIH pour les événements obligatoires de son cycle de vie.

15. Utilisation d'une composition pharmaceutique telle que définie dans l'une quelconque des revendications 1 à 11 pour la fabrication d'un médicament destiné au traitement ou à la prévention d'une infection virale choisie dans le groupe constitué des infections par le virus de l'immunodéficience du singe, le VIH-2, le HTLV-I et le HTLV-II.

16. Composition pharmaceutique telle que définie dans l'une quelconque des revendications 1 à 11 pour utilisation dans le traitement ou la prévention de maladies de type SIDA causées par un rétrovirus qui dépend des aspartyl-protéases similaires à celles présentes dans le VIH pour les événements obligatoires de son cycle de vie.

17. Composition pharmaceutique telle que définie dans l'une quelconque des revendications 1 à 11 pour utilisation dans le traitement ou la prévention d'une infection par le VIH.

18. Composition pharmaceutique telle que définie dans l'une quelconque des revendications 1 à 11 pour utilisation dans le traitement ou la prévention d'une infection virale choisie dans le groupe constitué des infections par le virus de l'immunodéficience du singe, le VIH-2, le HTLV-I et le HTLV-II.

19. Composition pharmaceutique selon la revendication 1, dans laquelle ledit agent antiviral contre le VIH est choisi parmi les polysaccharides polysulfatés, le sT4 (CD4 soluble) et autres composés qui bloquent la liaison du virus aux récepteurs de CD4 sur les lymphocytes T porteurs de CD4 et autres cellules de CD4(+), ou inhibent la fusion de l'enveloppe virale avec la membrane cytoplasmique, la didanosine (ddI), la zalcitabine (ddC), la stavudine (D4T), la zidovudine (AZT) et la lamivudine (3TC).
